(19) Europäisches Patentamt
European Patent Office
Office européen des brevets

(11) **EP 4 438 627 A1**

(12) **EUROPEAN PATENT APPLICATION**
published in accordance with Art. 153(4) EPC

(43) Date of publication:
**02.10.2024 Bulletin 2024/40**

(21) Application number: **22903502.7**

(22) Date of filing: **07.12.2022**

(51) International Patent Classification (IPC):
**C07K 16/30** (2006.01)     **A61P 35/00** (2006.01)
**A61K 39/395** (2006.01)

(52) Cooperative Patent Classification (CPC):
**A61K 39/395; A61P 35/00; C07K 16/30**

(86) International application number:
**PCT/CN2022/137192**

(87) International publication number:
**WO 2023/104080 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.12.2021 CN 202111482535**

(71) Applicant: **Chia Tai Tianqing Pharmaceutical Group Co., Ltd.**
**Lianyungang City, Jiangsu 222062 (CN)**

(72) Inventors:
• **ZHEN, Zipeng**
  **Nanjing City, Jiangsu 211100 (CN)**
• **HUANG, Liangmin**
  **Nanjing City, Jiangsu 211100 (CN)**
• **MA, Yuanjing**
  **Nanjing City, Jiangsu 211100 (CN)**
• **LV, Caiyun**
  **Nanjing City, Jiangsu 211100 (CN)**
• **ZHANG, Zhengping**
  **Nanjing City, Jiangsu 211100 (CN)**

(74) Representative: **Müller Schupfner & Partner Patent- und Rechtsanwaltspartnerschaft mbB (Muc)**
**Bavariaring 11**
**80336 München (DE)**

Remarks:
The complete document including Reference Table(s) and the Sequence Listing(s) can be downloaded from the EPO website

(54) **ANTI-TROP-2 ANTIBODY OR ANTIGEN-BINDING FRAGMENT THEREOF**

(57)     Provided are an anti-TROP-2 antibody or an antigen-binding fragment thereof, a pharmaceutical composition comprising same, and the uses of the anti-TROP-2 antibody or the antigen-binding fragment thereof or the pharmaceutical composition comprising same in the preparation of drugs for cancer treatment.

EP 4 438 627 A1

**Description**

**TECHNICAL FIELD**

The present application relates to an anti-TROP-2 antibody or an antigen-binding fragment thereof, a murine antibody, a chimeric antibody, and a humanized antibody comprising CDR regions of the anti-TROP-2 antibody, a nucleic acid encoding the same, a pharmaceutical composition comprising the same, and use of such antibodies or antigen-binding fragments for preparing a medicament for the treatment of cancer.

**BACKGROUND**

Human Trop-2, the full name of which is human trophoblast cell surface glycoprotein antigen 2, is also called tumor-associated calcium ion signal transducer 2 (TACSTD2), epidermal glycoprotein 1(EGP-1), gastrointestinal tumor-associated antigen (GA733-1), and surface marker 1(M1S1). It is a transmembrane cell surface glycoprotein encoded and expressed by Tacstd2 gene in chromosome 1p32 region, belongs to the GA733 protein family, and has a relatively high structural sequence similarity with epithelial cell adhesion molecules (EpCAM, also called Trop-1 and TACSTD1), with the homology reaching 49%.

The human Trop-2 protein (323 amino acid residues) has a molecular weight of about 36 kD and comprises a hydrophilic signal peptide (amino acids at positions 1-26), an extracellular domain (amino acids at positions 27-274), a cell transmembrane domain (amino acids at positions 275-297), and an intracellular domain (amino acids at positions 298-323). The extracellular domain is generally thought to be involved in the proliferation, infiltration, and metastasis of cancer cells.

Physiological ligands for human Trop-2 have not been identified so far, the molecular functions have not been fully elucidated, but it has been disclosed that it transmits calcium signals in tumor cells; the serine residue at position 303 in the Trop2 cell is phosphorylated by the protein kinase c (PKC), a $Ca^{2+}$-dependent protein kinase, and the intracellular domain has the PIP2 binding sequence, suggesting that it has signaling function in tumor cells.

It has been reported that human Trop-2 is overexpressed in various epithelial-derived cancers, such as gastric cancer, lung cancer, colorectal cancer, ovarian cancer, breast cancer, prostate cancer, and the like. In contrast, its expression in normal tissues is limited to cells in the epithelial region, and the expression level is lower than that in tumor cells. This suggests that it is involved in tumor formation. Therefore, the research on the anti-tumor drugs taking Trop-2 as a target is of great significance.

**SUMMARY**

The present application provides an isolated antibody or an antigen-binding fragment thereof binding to TROP-2 that features high affinity, high selectivity, and high bioactivity.

In one aspect, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3 in the variable region sequence set forth in SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, SEQ ID NO: 64, or SEQ ID NO:65, and the light chain variable region comprises a light chain CDR1, a light chain CDR2, and a light chain CDR3 in the variable region sequence set forth in SEQ ID NO: 24, SEQ ID NO: 26, SEQ ID NO: 28, SEQ ID NO: 40, SEQ ID NO: 41, SEQ ID NO: 54, SEQ ID NO: 55, SEQ ID NO: 66, or SEQ ID NO: 67.

Alternatively, the present application provides an isolated antibody or an antigen-binding fragment thereof binding to human TROP-2, which comprises:

a heavy chain CDR1, a heavy chain CDR2, and a heavy chain CDR3 contained in the heavy chain variable region sequence set forth in SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, SEQ ID NO: 64, or SEQ ID NO: 65, and/or
a light chain CDR1, a light chain CDR2, and a light chain CDR3 contained in the light chain variable region sequence set forth in SEQ ID NO: 24, SEQ ID NO: 26, SEQ ID NO: 28, SEQ ID NO: 40, SEQ ID NO: 41, SEQ ID NO: 54, SEQ ID NO: 55, SEQ ID NO: 66, or SEQ ID NO: 67.

In one aspect, the present application provides an antibody or an antigen-binding fragment thereof binding to human TROP-2, which comprises a heavy chain variable region and a light chain variable region, wherein: the heavy chain variable region comprises the heavy chain CDR1, the heavy chain CDR2, and the heavy chain CDR3 in the variable region sequence set forth in SEQ ID NO: 23, and the light chain variable region comprises the light chain CDR1, the light chain CDR2, and the light chain CDR3 in the variable region sequence set forth in SEQ ID NO: 24; the heavy chain variable region comprises the heavy chain CDR1, the heavy chain CDR2, and the heavy chain CDR3 in the variable region sequence set forth in SEQ ID NO: 25, and the light chain variable region comprises the light chain CDR1, the light chain CDR2, and the light chain CDR3 in the variable region sequence set forth in SEQ ID NO: 26; the heavy chain variable region comprises the heavy chain CDR1, the heavy chain CDR2, and the heavy chain CDR3 in the variable region sequence set forth in SEQ ID NO: 27, and the light chain variable region comprises the light chain CDR1, the light chain CDR2, and the light chain CDR3 in the variable region sequence set forth in SEQ ID NO: 28; the heavy chain variable region comprises the heavy chain CDR1, the heavy chain CDR2, and the heavy chain CDR3 in the variable region sequence set forth in SEQ ID NO: 36, and the light chain variable region comprises the light chain CDR1, the light chain CDR2, and the light chain CDR3 in the variable region sequence set forth in SEQ ID NO: 40; the heavy chain variable region comprises the heavy chain CDR1, the heavy chain CDR2, and the heavy chain CDR3 in the variable region sequence set forth in SEQ ID NO: 37, and the light chain variable region comprises the light chain CDR1, the light chain CDR2, and the light chain CDR3 in the variable region sequence set forth in SEQ ID NO: 40; the heavy chain variable region comprises the heavy chain CDR1, the heavy chain CDR2, and the heavy chain CDR3 in the variable region sequence set forth in SEQ ID NO: 38, and the light chain variable region comprises the light chain CDR1, the light chain CDR2, and the light chain CDR3 in the variable region sequence set forth in SEQ ID NO: 40; the heavy chain variable region comprises the heavy chain CDR1, the heavy chain CDR2, and the heavy chain CDR3 in the variable region sequence set forth in SEQ ID NO: 39, and the light chain variable region comprises the light chain CDR1, the light chain CDR2, and the light chain CDR3 in the variable region sequence set forth in SEQ ID NO: 40; the heavy chain variable region comprises the heavy chain CDR1, the heavy chain CDR2, and the heavy chain CDR3 in the variable region sequence set forth in SEQ ID NO: 36, and the light chain variable region comprises the light chain CDR1, the light chain CDR2, and the light chain CDR3 in the variable region sequence set forth in SEQ ID NO: 41; the heavy chain variable region comprises the heavy chain CDR1, the heavy chain CDR2, and the heavy chain CDR3 in the variable region sequence set forth in SEQ ID NO: 37, and the light chain variable region comprises the light chain CDR1, the light chain CDR2, and the light chain CDR3 in the variable region sequence set forth in SEQ ID NO: 41; the heavy chain variable region comprises the heavy chain CDR1, the heavy chain CDR2, and the heavy chain CDR3 in the variable region sequence set forth in SEQ ID NO: 38, and the light chain variable region comprises the light chain CDR1, the light chain CDR2, and the light chain CDR3 in the variable region sequence set forth in SEQ ID NO: 41; the heavy chain variable region comprises the heavy chain CDR1, the heavy chain CDR2, and the heavy chain CDR3 in the variable region sequence set forth in SEQ ID NO: 39, and the light chain variable region comprises the light chain CDR1, the light chain CDR2, and the light chain CDR3 in the variable region sequence set forth in SEQ ID NO: 41; the heavy chain variable region comprises the heavy chain CDR1, the heavy chain CDR2, and the heavy chain CDR3 in the variable region sequence set forth in SEQ ID NO: 50, and the light chain variable region comprises the light chain CDR1, the light chain CDR2, and the light chain CDR3 in the variable region sequence set forth in SEQ ID NO: 54; the heavy chain variable region comprises the heavy chain CDR1, the heavy chain CDR2, and the heavy chain CDR3 in the variable region sequence set forth in SEQ ID NO: 51, and the light chain variable region comprises the light chain CDR1, the light chain CDR2, and the light chain CDR3 in the variable region sequence set forth in SEQ ID NO: 54; the heavy chain variable region comprises the heavy chain CDR1, the heavy chain CDR2, and the heavy chain CDR3 in the variable region sequence set forth in SEQ ID NO: 52, and the light chain variable region comprises the light chain CDR1, the light chain CDR2, and the light chain CDR3 in the variable region sequence set forth in SEQ ID NO: 54; the heavy chain variable region comprises the heavy chain CDR1, the heavy chain CDR2, and the heavy chain CDR3 in the variable region sequence set forth in SEQ ID NO: 53, and the light chain variable region comprises the light chain CDR1, the light chain CDR2, and the light chain CDR3 in the variable region sequence set forth in SEQ ID NO: 54; the heavy chain variable region comprises the heavy chain CDR1, the heavy chain CDR2, and the heavy chain CDR3 in the variable region sequence set forth in SEQ ID NO: 50, and the light chain variable region comprises the light chain CDR1, the light chain CDR2, and the light chain CDR3 in the variable region sequence set forth in SEQ ID NO: 55; the heavy chain variable region comprises the heavy chain CDR1, the heavy chain CDR2, and the heavy chain CDR3 in the variable region sequence set forth in SEQ ID NO: 51, and the light chain variable region comprises the light chain CDR1, the light chain CDR2, and the light chain CDR3 in the variable region sequence set forth in SEQ ID NO: 55; the heavy chain variable region comprises the heavy chain CDR1, the heavy chain CDR2, and the heavy chain CDR3 in the variable region sequence set forth in SEQ ID NO: 52, and the light chain variable region comprises the light chain CDR1, the light chain CDR2, and the light chain CDR3 in the variable region sequence set forth in SEQ ID NO: 55; the heavy chain variable region comprises the heavy chain CDR1, the heavy chain CDR2, and the heavy chain CDR3 in the variable region sequence set forth in SEQ ID NO: 53, and the light chain variable region comprises the light chain CDR1, the light chain CDR2, and the light chain CDR3 in the variable region sequence set forth in SEQ ID NO: 55; the heavy chain variable region comprises the heavy chain CDR1, the heavy chain CDR2, and the heavy chain CDR3 in the variable region sequence set forth in SEQ ID NO: 63, and the light chain variable region comprises the light chain CDR1, the light chain CDR2, and the light chain CDR3 in the variable region sequence set forth in SEQ ID NO: 66; the heavy chain variable region comprises the heavy chain CDR1, the heavy chain CDR2, and the heavy chain CDR3 in the variable region sequence set forth in SEQ ID NO: 64, and the light chain variable region comprises the light chain CDR1, the light chain CDR2, and the light chain CDR3 in the variable region sequence set forth in SEQ ID NO: 66; the heavy chain variable region comprises the heavy chain CDR1, the heavy chain CDR2, and the heavy chain CDR3 in the variable region sequence set forth in SEQ ID NO: 65, and the light chain variable region comprises the light chain CDR1, the light chain CDR2, and the light chain CDR3 in the variable region sequence set forth in SEQ ID NO: 66; the heavy chain variable region comprises the heavy chain CDR1, the heavy chain CDR2, and the heavy chain CDR3 in the variable region sequence set forth in SEQ ID NO: 63, and the light chain variable region comprises the light chain CDR1, the light chain CDR2, and the light chain CDR3 in the variable region sequence set forth in SEQ ID NO: 67; the heavy chain variable region comprises the heavy chain CDR1, the heavy chain CDR2, and the heavy chain CDR3 in the variable region sequence set forth in SEQ ID NO: 64, and the light chain variable region comprises the light chain CDR1, the light chain CDR2, and the light chain CDR3 in the variable region sequence set forth in SEQ ID NO: 67; or the heavy chain variable region comprises the heavy chain CDR1, the heavy chain CDR2, and the heavy chain CDR3 in the variable region sequence set forth in SEQ ID NO: 65, and the light chain variable region comprises the light chain CDR1, the light chain CDR2, and the light chain CDR3 in the variable region sequence set forth in SEQ ID NO: 67.

In one aspect, the present application provides an antibody or an antigen-binding fragment thereof binding to human TROP-2, which comprises a heavy chain variable region and a light chain variable region, wherein: the heavy chain variable region comprises a heavy chain CDR1, a heavy chain CDR2, and a heavy chain CDR3, the heavy chain CDR1 having at least 80% identity to an amino acid sequence selected from the group consisting of SEQ ID NO: 5, SEQ ID NO: 11, SEQ ID NO: 48, and SEQ ID NO: 17, the heavy chain CDR2 having at least 80% identity to an amino acid sequence selected from the group consisting of SEQ ID NO: 6, SEQ ID NO: 31, SEQ ID NO: 32, SEQ ID NO: 49, SEQ ID NO: 12, SEQ ID NO: 62, and SEQ ID NO: 18, and the heavy chain CDR3 having at least 80% identity to an amino acid sequence selected from the group consisting of SEQ ID NO: 7, SEQ ID NO: 13, and SEQ ID NO: 19; the light chain variable region comprises a light chain CDR1, a light chain CDR2, and a light chain CDR3, the light chain CDR1 having at least 80% identity to an amino acid sequence selected from the group consisting of SEQ ID NO: 8, SEQ ID NO: 33, SEQ ID NO: 14, and SEQ ID NO: 20, the light chain CDR2 having at least 80% identity to an amino acid sequence selected from the group consisting of SEQ ID NO: 9, SEQ ID NO: 34, SEQ ID NO: 15, and SEQ ID NO: 21, and the light chain CDR3 having at least 80% identity to an amino acid sequence selected from the group consisting of SEQ ID NO: 10, SEQ ID NO: 35, SEQ ID NO: 16, and SEQ ID NO: 22.

Alternatively, the present application provides an isolated antibody or an antigen-binding fragment thereof binding to human TROP-2, which comprises:

a heavy chain CDR1, a heavy chain CDR2, and a heavy chain CDR3, the heavy chain CDR1 comprising the amino acid sequence set forth in SEQ ID NO: 5, SEQ ID NO: 11, SEQ ID NO: 48 or SEQ ID NO: 17 or a sequence having at least 80% identity thereto, the heavy chain CDR2 comprising the amino acid sequence set forth in SEQ ID NO: 6, SEQ ID NO: 31, SEQ ID NO: 32, SEQ ID NO: 49, SEQ ID NO: 12, SEQ ID NO: 62 or SEQ ID NO: 18 or a sequence having at least 80% identity thereto, and the heavy chain CDR3 comprising the amino acid sequence set forth in SEQ ID NO: 7, SEQ ID NO: 13 or SEQ ID NO. 19 or a sequence having at least 80% identity thereto; and/or

a light chain CDR1, a light chain CDR2, and a light chain CDR3, the light chain CDR1 comprising the amino acid sequence set forth in SEQ ID NO: 8, SEQ ID NO: 33, SEQ ID NO: 14 or SEQ ID NO: 20 or a sequence having at least 80% identity thereto, the light chain CDR2 comprising the amino acid sequence set forth in SEQ ID NO: 9, SEQ ID NO: 34, SEQ ID NO: 15 or SEQ ID NO: 21 or a sequence having at least 80% identity thereto, and the light chain CDR3 comprising the amino acid sequence set forth in SEQ ID NO: 10, SEQ ID NO: 35, SEQ ID NO: 16 or SEQ ID NO: 22 or a sequence having at least 80% identity thereto.

In one aspect, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, the heavy chain CDR1 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 5, SEQ ID NO: 11, SEQ ID NO: 48, and SEQ ID NO: 17.

In one aspect, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, the heavy chain CDR2 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 6, SEQ ID NO: 31, SEQ ID NO: 32, SEQ ID NO: 49, SEQ ID NO: 12, SEQ ID NO: 62, and SEQ ID NO: 18.

In one aspect, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, the heavy chain CDR3 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 7, SEQ ID NO: 13, and SEQ ID NO: 19.

In one aspect, the present application provides an antibody or an antigen-binding fragment thereof binding to human TROP-2, which comprises a heavy chain variable region and a light chain variable region, wherein the light chain variable region comprises a light chain CDR1, a light chain CDR2, and a light chain CDR3, the light chain CDR1 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 8, SEQ ID NO: 33, SEQ ID NO: 14, and SEQ ID NO: 20.

In one aspect, the present application provides an antibody or an antigen-binding fragment thereof binding to human TROP-2, which comprises a heavy chain variable region and a light chain variable region, wherein the light chain variable region comprises a light chain CDR1, a light chain CDR2, and a light chain CDR3, the light chain CDR2 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 9, SEQ ID NO: 34, SEQ ID NO: 15, and SEQ ID NO: 21.

In one aspect, the present application provides an antibody or an antigen-binding fragment thereof binding to human TROP-2, which comprises a heavy chain variable region and a light chain variable region, wherein the light chain variable region comprises a light chain CDR1, a light chain CDR2, and a light chain CDR3, the light chain CDR3 having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% sequence identity to an amino acid sequence selected from the group consisting of SEQ ID NO: 10, SEQ ID NO: 35, SEQ ID NO: 16, and SEQ ID NO: 22.

In one aspect, the present application provides an antibody or an antigen-binding fragment thereof binding to human TROP-2, which comprises a heavy chain variable region and a light chain variable region, wherein: the heavy chain variable region comprises a heavy chain CDR1, a heavy chain CDR2, and a heavy chain CDR3, the heavy chain CDR1 being an amino acid sequence selected from the group consisting of SEQ ID NO: 5, SEQ ID NO: 11, SEQ ID NO: 48, and SEQ ID NO: 17, the heavy chain CDR2 being an amino acid sequence selected from the group consisting of SEQ ID NO: 6, SEQ ID NO: 31, SEQ ID NO: 32, SEQ ID NO: 49, SEQ ID NO: 12, SEQ ID NO: 62, and SEQ ID NO: 18, and the heavy chain CDR3 being an amino acid sequence selected from the group consisting of SEQ ID NO: 7, SEQ ID NO: 13, and SEQ ID NO: 19; the light chain variable region comprises a light chain CDR1, a light chain CDR2, and a light chain CDR3, the light chain CDR1 being an amino acid sequence selected from the group consisting of SEQ ID NO: 8, SEQ ID NO: 33, SEQ ID NO: 14, and SEQ ID NO: 20, the light chain CDR2 being an amino acid sequence selected from the group consisting of SEQ ID NO: 9, SEQ ID NO: 34, SEQ ID NO: 15, and SEQ ID NO: 21, and the light chain CDR3 being an amino acid sequence selected from the group consisting of SEQ ID NO: 10, SEQ ID NO: 35, SEQ ID NO: 16, and SEQ ID NO: 22.

In one aspect, the present application provides an isolated antibody or an antigen-binding fragment thereof binding to human TROP-2, which comprises:

a heavy chain CDR1, a heavy chain CDR2, and a heavy chain CDR3, the heavy chain CDR1 having the amino acid sequence set forth in SEQ ID NO: 48 or 17, the heavy chain CDR2 having the amino acid sequence set forth in SEQ ID NO: 49, 18, or 62, and the heavy chain CDR3 having the amino acid sequence set forth in SEQ ID NO: 13 or 19; and
a light chain CDR1, a light chain CDR2, and a light chain CDR3, the light chain CDR1 having the amino acid sequence

EP 4 438 627 A1

set forth in SEQ ID NO: 14 or 20, the light chain CDR2 having the amino acid sequence set forth in SEQ ID NO: 15 or 21, and the light chain CDR3 having the amino acid sequence set forth in SEQ ID NO: 16 or 22.

In some embodiments, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, and the light chain variable region comprises a light chain CDR1, a light chain CDR2, and a light chain CDR3, wherein the heavy chain CDR1, the heavy chain CDR2, the heavy chain CDR3, the light chain CDR1, the light chain CDR2, and the light chain CDR3 are selected from the group consisting of the following sequences or sequences having at least 80% identity thereto, respectively:

| | | |
|---|---|---|
| i. | heavy chain CDR1 | SEQ ID NO: 5 |
| | heavy chain CDR2 | SEQ ID NO: 31 |
| | heavy chain CDR3 | SEQ ID NO: 7 |
| | light chain CDR1 | SEQ ID NO: 8 |
| | light chain CDR2 | SEQ ID NO: 34 |
| | light chain CDR3 | SEQ ID NO: 10; or, |
| ii. | heavy chain CDR1 | SEQ ID NO: 5 |
| | heavy chain CDR2 | SEQ ID NO: 32 |
| | heavy chain CDR3 | SEQ ID NO: 7 |
| | light chain CDR1 | SEQ ID NO: 8 |
| | light chain CDR2 | SEQ ID NO: 34 |
| | light chain CDR3 | SEQ ID NO: 10; or, |
| iii. | heavy chain CDR1 | SEQ ID NO: 5 |
| | heavy chain CDR2 | SEQ ID NO: 31 |
| | heavy chain CDR3 | SEQ ID NO: 7 |
| | light chain CDR1 | SEQ ID NO: 33 |
| | light chain CDR2 | SEQ ID NO: 34 |
| | light chain CDR3 | SEQ ID NO: 35; or, |
| iv. | heavy chain CDR1 | SEQ ID NO: 5 |
| | heavy chain CDR2 | SEQ ID NO: 32 |
| | heavy chain CDR3 | SEQ ID NO: 7 |
| | light chain CDR1 | SEQ ID NO: 33 |
| | light chain CDR2 | SEQ ID NO: 34 |
| | light chain CDR3 | SEQ ID NO: 35; or, |
| v. | heavy chain CDR1 | SEQ ID NO: 48 |
| | heavy chain CDR2 | SEQ ID NO: 49 |
| | heavy chain CDR3 | SEQ ID NO: 13 |
| | light chain CDR1 | SEQ ID NO: 14 |
| | light chain CDR2 | SEQ ID NO: 15 |
| | light chain CDR3 | SEQ ID NO: 16; or, |
| vi. | heavy chain CDR1 | SEQ ID NO: 48 |
| | heavy chain CDR2 | SEQ ID NO: 12 |
| | heavy chain CDR3 | SEQ ID NO: 13 |
| | light chain CDR1 | SEQ ID NO: 14 |
| | light chain CDR2 | SEQ ID NO: 15 |
| | light chain CDR3 | SEQ ID NO: 16; or, |
| vii. | heavy chain CDR1 | SEQ ID NO: 17 |
| | heavy chain CDR2 | SEQ ID NO: 62 |
| | heavy chain CDR3 | SEQ ID NO: 19 |
| | light chain CDR1 | SEQ ID NO: 20 |
| | light chain CDR2 | SEQ ID NO: 21 |
| | light chain CDR3 | SEQ ID NO: 22; or, |

6

(continued)

| | | | |
|---|---|---|---|
| viii. | heavy chain CDR1 | SEQ ID NO: 17 |
| | heavy chain CDR2 | SEQ ID NO: 18 |
| | heavy chain CDR3 | SEQ ID NO: 19 |
| | light chain CDR1 | SEQ ID NO: 20 |
| | light chain CDR2 | SEQ ID NO: 21 |
| | light chain CDR3 | SEQ ID NO: 22; or, |
| ix. | heavy chain CDR1 | SEQ ID NO: 5 |
| | heavy chain CDR2 | SEQ ID NO: 6 |
| | heavy chain CDR3 | SEQ ID NO: 7 |
| | light chain CDR1 | SEQ ID NO: 8 |
| | light chain CDR2 | SEQ ID NO: 9 |
| | light chain CDR3 | SEQ ID NO: 10; or, |
| x. | heavy chain CDR1 | SEQ ID NO: 11 |
| | heavy chain CDR2 | SEQ ID NO: 12 |
| | heavy chain CDR3 | SEQ ID NO: 13 |
| | light chain CDR1 | SEQ ID NO: 14 |
| | light chain CDR2 | SEQ ID NO: 15 |
| | light chain CDR3 | SEQ ID NO: 16; or, |
| xi. | heavy chain CDR1 | SEQ ID NO: 17 |
| | heavy chain CDR2 | SEQ ID NO: 18 |
| | heavy chain CDR3 | SEQ ID NO: 19 |
| | light chain CDR1 | SEQ ID NO: 20 |
| | light chain CDR2 | SEQ ID NO: 21 |
| | light chain CDR3 | SEQ ID NO: 22. |

In one aspect, the present application provides an antibody or an antigen-binding fragment thereof binding to human TROP-2, which comprises a heavy chain variable region and a light chain variable region, wherein the heavy chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, SEQ ID NO: 64, and SEQ ID NO: 65, and wherein the light chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 24, SEQ ID NO: 26, SEQ ID NO: 28, SEQ ID NO: 40, SEQ ID NO: 41, SEQ ID NO: 54, SEQ ID NO: 55, SEQ ID NO: 66, and SEQ ID NO: 67.

Alternatively, the present application provides an isolated antibody or an antigen-binding fragment thereof binding to human TROP-2, which comprises:

a heavy chain variable region comprising the amino acid sequence set forth in SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, SEQ ID NO: 64 or SEQ ID NO: 65 or an amino acid sequence having at least 80% identity thereto; and/or
a light chain variable region comprising the amino acid sequence set forth in SEQ ID NO: 24, SEQ ID NO: 26, SEQ ID NO: 28, SEQ ID NO: 40, SEQ ID NO: 41, SEQ ID NO: 54, SEQ ID NO: 55, SEQ ID NO: 66 or SEQ ID NO: 67 or an amino acid sequence having at least 80% identity thereto.

In one aspect, the present application provides an antibody or an antigen-binding fragment thereof binding to human TROP-2, which comprises a heavy chain variable region and a light chain variable region, wherein the heavy chain variable region is an amino acid sequence selected from the group consisting of SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, SEQ ID NO: 64, and SEQ ID NO:65, and the light chain variable region is an amino acid sequence selected from the group consisting of SEQ ID NO: 24, SEQ ID NO: 26, SEQ ID NO: 28, SEQ ID NO: 40, SEQ ID NO: 41, SEQ ID NO: 54, SEQ ID NO: 55, SEQ ID NO: 66, and SEQ ID NO: 67.

In one aspect, the present application provides an isolated antibody or an antigen-binding fragment thereof binding to human TROP-2, which comprises:

a heavy chain variable region having the amino acid sequence set forth in SEQ ID NO: 50, 63, 64, or 65; and
a light chain variable region having the amino acid sequence set forth in SEQ ID NO: 55, 66, or 67.

In one aspect, the present application provides an antibody or an antigen-binding fragment thereof binding to human TROP-2, which comprises a heavy chain variable region and a light chain variable region, wherein the heavy chain variable region and the light chain variable region are selected from the group consisting of the following sequences or sequences having at least 80% identity thereto, respectively:

i. heavy chain variable region SEQ ID NO: 36, light chain variable region SEQ ID NO: 40; or,
ii. heavy chain variable region SEQ ID NO: 37, light chain variable region SEQ ID NO: 40; or,
iii. heavy chain variable region SEQ ID NO: 38, light chain variable region SEQ ID NO: 40; or,
iv. heavy chain variable region SEQ ID NO: 39, light chain variable region SEQ ID NO: 40; or,
v. heavy chain variable region SEQ ID NO: 36, light chain variable region SEQ ID NO: 41; or,
vi. heavy chain variable region SEQ ID NO: 37, light chain variable region SEQ ID NO: 41; or,
vii. heavy chain variable region SEQ ID NO: 38, light chain variable region SEQ ID NO: 41; or,
viii. heavy chain variable region SEQ ID NO: 39, light chain variable region SEQ ID NO: 41; or,
ix. heavy chain variable region SEQ ID NO: 50, light chain variable region SEQ ID NO: 54; or,
x. heavy chain variable region SEQ ID NO: 51, light chain variable region SEQ ID NO: 54; or,
xi. heavy chain variable region SEQ ID NO: 52, light chain variable region SEQ ID NO: 54; or,
xii. heavy chain variable region SEQ ID NO: 53, light chain variable region SEQ ID NO: 54; or,
xiii. heavy chain variable region SEQ ID NO: 50, light chain variable region SEQ ID NO: 55; or,
xiv. heavy chain variable region SEQ ID NO: 51, light chain variable region SEQ ID NO: 55; or,
xv. heavy chain variable region SEQ ID NO: 52, light chain variable region SEQ ID NO: 55; or,
xvi. heavy chain variable region SEQ ID NO: 53, light chain variable region SEQ ID NO: 55; or,
xvii. heavy chain variable region SEQ ID NO: 63, light chain variable region SEQ ID NO: 66; or,
xviii. heavy chain variable region SEQ ID NO: 64, light chain variable region SEQ ID NO: 66; or,
xix. heavy chain variable region SEQ ID NO: 65, light chain variable region SEQ ID NO: 66; or,
xx. heavy chain variable region SEQ ID NO: 63, light chain variable region SEQ ID NO: 67; or,
xxi. heavy chain variable region SEQ ID NO: 64, light chain variable region SEQ ID NO: 67; or,
xxii. heavy chain variable region SEQ ID NO: 65, light chain variable region SEQ ID NO: 67; or,
xxiii. heavy chain variable region SEQ ID NO: 23, light chain variable region SEQ ID NO: 24; or,
xxiv. heavy chain variable region SEQ ID NO: 25, light chain variable region SEQ ID NO: 26; or,
xxv. heavy chain variable region SEQ ID NO: 27, light chain variable region SEQ ID NO: 28.

In some embodiments, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, and the light chain variable region comprises a light chain CDR1, a light chain CDR2, and a light chain CDR3, wherein the heavy chain CDR1 comprises the amino acid sequence set forth in SEQ ID NO: 48, the heavy chain CDR2 comprises the amino acid sequence set forth in SEQ ID NO: 49, the heavy chain CDR3 comprises the amino acid sequence set forth in SEQ ID NO: 13, the light chain CDR1 comprises the amino acid sequence set forth in SEQ ID NO: 14, the light chain CDR2 comprises the amino acid sequence set forth in SEQ ID NO: 15, and the light chain CDR3 comprises the amino acid sequence set forth in SEQ ID NO: 16; the heavy chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 50, and the light chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 55.

In some embodiments, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, and the light chain variable region comprises a light chain CDR1, a light chain CDR2, and a light chain CDR3, wherein the heavy chain CDR1 comprises the amino acid sequence set forth in SEQ ID NO: 17, the heavy chain CDR2 comprises the amino acid sequence set forth in SEQ ID NO: 18, the heavy chain CDR3 comprises the amino acid sequence set forth in SEQ ID NO: 19, the light chain CDR1 comprises the amino acid sequence set forth in SEQ ID NO: 20, the light chain CDR2 comprises the amino acid sequence set forth in SEQ ID NO: 21, and the light chain CDR3 comprises the amino acid sequence set forth in SEQ ID NO: 22; the heavy chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 65, and the light chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 66.

In some embodiments, the present application provides an antibody or an antigen-binding fragment thereof binding to human TROP-2, which comprises a heavy chain variable region and a light chain variable region, wherein the heavy chain variable region comprises a heavy chain CDR1, a heavy chain CDR2, and a heavy chain CDR3, and the light chain variable region comprises a light chain CDR1, a light chain CDR2, and a light chain CDR3, wherein the heavy chain CDR1 comprises the amino acid sequence set forth in SEQ ID NO: 17, the heavy chain CDR2 comprises the amino acid sequence set forth in SEQ ID NO: 18, the heavy chain CDR3 comprises the amino acid sequence set forth in SEQ ID NO: 19, the light chain CDR1 comprises the amino acid sequence set forth in SEQ ID NO: 20, the light chain CDR2 comprises the amino acid sequence set forth in SEQ ID NO: 21, and the light chain CDR3 comprises the amino acid sequence set forth in SEQ ID NO: 22; the heavy chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 65, and the light chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 67.

In some embodiments, the antibody or the antigen-binding fragment thereof binding to human TROP-2 provided by the present application is a murine antibody, a chimeric antibody, a humanized antibody, or a fully human antibody.

In one aspect, the present application provides an antibody or an antigen-binding fragment thereof binding to human TROP-2, wherein the TROP-2 antibody comprises a heavy chain constant region and a light chain constant region; preferably, the heavy chain constant region is selected from the group consisting of constant regions of human IgG1, IgG2, IgG3, and IgG4, and the light chain constant region selected from the group consisting of constant regions of human κ and λ chains; more preferably, the antibody comprises a heavy chain constant region having the sequence set forth in SEQ ID NO: 29 and a light chain constant region having the sequence set forth in SEQ ID NO: 30.

In some embodiments, the present application provides a chimeric antibody binding to human TROP-2, which comprises a heavy chain and a light chain, wherein the heavy chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 73, SEQ ID NO: 75, and SEQ ID NO: 77, and wherein the light chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 74, SEQ ID NO: 76, and SEQ ID NO: 78.

In some embodiments, the present application provides a humanized antibody binding to human TROP-2, which comprises a heavy chain and a light chain, wherein the heavy chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 42, SEQ ID NO: 43, SEQ ID NO: 44, SEQ ID NO: 45, SEQ ID NO: 56, SEQ ID NO: 57, SEQ ID NO: 58, SEQ ID NO: 59, SEQ ID NO: 68, SEQ ID NO: 69, and SEQ ID NO: 70, and wherein the light chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 46, SEQ ID NO: 47, SEQ ID NO: 60, SEQ ID NO: 61, SEQ ID NO: 71, and SEQ ID NO: 72.

Alternatively, the present application provides an isolated antibody or an antigen-binding fragment thereof binding to human TROP-2, which comprises:

a heavy chain comprising the amino acid sequence set forth in SEQ ID NO: 73, SEQ ID NO: 75, SEQ ID NO: 77, SEQ ID NO: 42, SEQ ID NO: 43, SEQ ID NO: 44, SEQ ID NO: 45, SEQ ID NO: 56, SEQ ID NO: 57, SEQ ID NO: 58, SEQ ID NO: 59, SEQ ID NO: 68, SEQ ID NO: 69 or SEQ ID NO: 70 or an amino acid sequence having at least 80% identity thereto; and/or
a light chain comprising the amino acid sequence set forth in SEQ ID NO: 74, SEQ ID NO: 76, SEQ ID NO: 78, SEQ ID NO: 46, SEQ ID NO: 47, SEQ ID NO: 60, SEQ ID NO: 61, SEQ ID NO: 71 or SEQ ID NO: 72 or an amino acid sequence having at least 80% identity thereto.

In some embodiments, the present application provides a humanized antibody binding to human TROP-2, which comprises a heavy chain and a light chain, wherein the heavy chain is selected from the group consisting of the amino acid sequences set forth in SEQ ID NO: 42, SEQ ID NO: 43, SEQ ID NO: 44, SEQ ID NO: 45, SEQ ID NO: 56, SEQ ID NO: 57, SEQ ID NO: 58, SEQ ID NO: 59, SEQ ID NO: 68, SEQ ID NO: 69, and SEQ ID NO: 70, and wherein the light chain is selected from the group consisting of the amino acid sequences set forth in SEQ ID NO: 46, SEQ ID NO: 47, SEQ ID NO: 60, SEQ ID NO: 61, SEQ ID NO: 71, and SEQ ID NO: 72.

In some embodiments, the present application provides an isolated humanized antibody or an antigen-binding fragment thereof binding to human TROP-2, which comprises:

a heavy chain having the amino acid sequence set forth in SEQ ID NO: 56, 68, 69, or 70, and
a light chain having the amino acid sequence set forth in SEQ ID NO: 61, 71, or 72.

In some embodiments, the present application provides a humanized antibody or an antigen-binding fragment thereof binding to human TROP-2, which comprises a heavy chain and a light chain, wherein the heavy chain and the light chain are selected from the group consisting of the following sequences or sequences having at least 80% identity thereto, respectively:

i) heavy chain SEQ ID NO: 42, light chain SEQ ID NO: 46; or,
ii) heavy chain SEQ ID NO: 43, light chain SEQ ID NO: 46; or,
iii) heavy chain SEQ ID NO: 44, light chain SEQ ID NO: 46; or,
iv) heavy chain SEQ ID NO: 45, light chain SEQ ID NO: 46; or,
v) heavy chain SEQ ID NO: 42, light chain SEQ ID NO: 47; or,
vi) heavy chain SEQ ID NO: 43, light chain SEQ ID NO: 47; or,
vii) heavy chain SEQ ID NO: 44, light chain SEQ ID NO: 47; or,
viii) heavy chain SEQ ID NO: 45, light chain SEQ ID NO: 47; or,
ix) heavy chain SEQ ID NO: 56, light chain SEQ ID NO: 60; or,
x) heavy chain SEQ ID NO: 57, light chain SEQ ID NO: 60; or,
xi) heavy chain SEQ ID NO: 58, light chain SEQ ID NO: 60; or,
xii) heavy chain SEQ ID NO: 59, light chain SEQ ID NO: 60; or,
xiii) heavy chain SEQ ID NO: 56, light chain SEQ ID NO: 61; or,
xiv) heavy chain SEQ ID NO: 57, light chain SEQ ID NO: 61; or,
xv) heavy chain SEQ ID NO: 58, light chain SEQ ID NO: 61; or,
xvi) heavy chain SEQ ID NO: 59, light chain SEQ ID NO: 61; or,
xvii) heavy chain SEQ ID NO: 68, light chain SEQ ID NO: 71; or,

xviii) heavy chain SEQ ID NO: 69, light chain SEQ ID NO: 71; or,
xix) heavy chain SEQ ID NO: 70, light chain SEQ ID NO: 71; or,
xx) heavy chain SEQ ID NO: 68, light chain SEQ ID NO: 72; or,
xxi) heavy chain SEQ ID NO: 69, light chain SEQ ID NO: 72; or,
xxii) heavy chain SEQ ID NO: 70, light chain SEQ ID NO: 72.

In some embodiments, the present application provides an antibody or an antigen-binding fragment thereof binding to human TROP-2, which comprises a heavy chain and a light chain, wherein the heavy chain comprises a heavy chain CDR1, a heavy chain CDR2, and a heavy chain CDR3, and the light chain comprises a light chain CDR1, a light chain CDR2, and a light chain CDR3, wherein the heavy chain CDR1 comprises the amino acid sequence set forth in SEQ ID NO: 48, the heavy chain CDR2 comprises the amino acid sequence set forth in SEQ ID NO: 49, the heavy chain CDR3 comprises the amino acid sequence set forth in SEQ ID NO: 13, the light chain CDR1 comprises the amino acid sequence set forth in SEQ ID NO: 14, the light chain CDR2 comprises the amino acid sequence set forth in SEQ ID NO: 15, and the light chain CDR3 comprises the amino acid sequence set forth in SEQ ID NO: 16; the heavy chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 56, and the light chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 61.

In some embodiments, the present application provides an antibody or an antigen-binding fragment thereof binding to human TROP-2, which comprises a heavy chain and a light chain, wherein the heavy chain comprises a heavy chain CDR1, a heavy chain CDR2, and a heavy chain CDR3, and the light chain comprises a light chain CDR1, a light chain CDR2, and a light chain CDR3, wherein the heavy chain CDR1 comprises the amino acid sequence set forth in SEQ ID NO: 17, the heavy chain CDR2 comprises the amino acid sequence set forth in SEQ ID NO: 18, the heavy chain CDR3 comprises the amino acid sequence set forth in SEQ ID NO: 19, the light chain CDR1 comprises the amino acid sequence set forth in SEQ ID NO: 20, the light chain CDR2 comprises the amino acid sequence set forth in SEQ ID NO: 21, and the light chain CDR3 comprises the amino acid sequence set forth in SEQ ID NO: 22; the heavy chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 70, and the light chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 71.

In some embodiments, the present application provides an antibody or an antigen-binding fragment thereof binding to human TROP-2, which comprises a heavy chain and a light chain, wherein the heavy chain comprises a heavy chain CDR1, a heavy chain CDR2, and a heavy chain CDR3, and the light chain comprises a light chain CDR1, a light chain CDR2, and a light chain CDR3, wherein the heavy chain CDR1 comprises the amino acid sequence set forth in SEQ ID NO: 17, the heavy chain CDR2 comprises the amino acid sequence set forth in SEQ ID NO: 18, the heavy chain CDR3 comprises the amino acid sequence set forth in SEQ ID NO: 19, the light chain CDR1 comprises the amino acid sequence set forth in SEQ ID NO: 20, the light chain CDR2 comprises the amino acid sequence set forth in SEQ ID NO: 21, and the light chain CDR3 comprises the amino acid sequence set forth in SEQ ID NO: 22; the heavy chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 70, and the light chain has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to the amino acid sequence set forth in SEQ ID NO: 72.

In some embodiments, the antibody or the antigen-binding fragment thereof provided by the present application is selected from the group consisting of a monoclonal antibody, a fusion protein, a multispecific antibody, a Fab fragment, a Fab' fragment, a F(ab')2 fragment, an Fd fragment, an Fv fragment, a dAb fragment, an isolated CDR region, an scFv, and a nanobody.

In some embodiments, the antibody or the antigen-binding fragment thereof provided by the present application is of the IgG1, IgG2, IgG3, or IgG4 type.

In one aspect, the present application provides an isolated antibody or an antigen-binding fragment thereof binding to Trop-2, wherein the antibody or the antigen-binding fragment thereof exhibits one of or a combination of more of the following properties:

(a) binding to Trop-2 with a $K_D$ value of 1.00E-08 M or less;
(b) blocking binding of Trop-2 to the ligand;
(c) exhibiting internalization activity in cells expressing Trop-2;
(d) inducing antibody-dependent cell-mediated cytotoxicity (ADCC) against cells expressing Trop-2;
(e) not binding to tumor cells that do not express Trop-2.

In another aspect, the present application provides an isolated antibody or an antigen-binding fragment thereof, wherein the antibody or the antigen-binding fragment thereof binds to the same epitope on Trop-2 as any of the exemplary antibodies or antigen-binding fragments thereof provided herein.

In some embodiments, the present application provides an antibody or an antigen-binding fragment thereof binding to TROP-2 that binds to the same epitope on TROP-2 as any of the exemplary antibodies provided herein, e.g., binds to the same epitope as the hz-Ab2 series of antibodies, e.g., binds to the same epitope as the hz-Ab21 series of antibodies, e.g., binds to the same epitope as the hz-Ab35 series of antibodies. In some embodiments, the antibody or the antigen-binding fragment thereof provided by the present application competes for binding to TROP-2 with any of the exemplary antibodies for binding to TROP-2 provided herein, e.g., competes for binding to TROP-2 with the hz-Ab2 series of antibodies, e.g., competes for binding to TROP-2 with the hz-Ab21 series of antibodies, e.g., competes for binding to TROP-2 with the hz-Ab35 series of antibodies. The binding to TROP-2 can be measured by ELISA, flow cytometry, surface plasmon resonance (SPR) assay, or any other methods known in the art.

In some embodiments, the heavy chain variable region and the light chain variable region described herein comprise the sequence characteristics of the heavy chain CDR1, the heavy chain CDR2, the heavy chain CDR3, the light chain CDR1, the light chain CDR2, and the light chain CDR3 described herein.

In some embodiments, the isolated antibody binding to Trop-2 provided herein comprises a heavy chain and a light chain that comprise constant regions in addition to the heavy chain variable region and the light chain variable region described herein. In some embodiments, the constant regions are humanized. In some embodiments, the framework regions (FRs) of the constant regions and the variable regions are humanized. Immunogenicity is reduced by constructing chimeric antibodies (e.g., humanized constant regions and non-human variable regions) or humanized antibodies (e.g., humanized constant regions and FRs).

In some embodiments, the light chain constant region of the antibody is a human κ chain constant region. In some embodiments, the light chain constant region of the antibody is a human λ chain constant region.

The heavy chain constant region of the antibody may be derived from a constant region of any type, e.g., IgG, IgM, IgD, IgA, and IgE, and of any isotype, e.g., IgG1, IgG2, IgG3, and IgG4. In some embodiments, the isolated antibody binding to Trop-2 is of the IgG1 isotype. In some embodiments, the isolated antibody binding to Trop-2 is of the IgG4 isotype.

In some embodiments, the antibody comprises a modified constant region. In some embodiments, the hinge region within the human IgG4 constant region is modified to avoid or reduce chain swapping; for example, an IgG4 antibody has a Ser228Pro (S228P) mutation according to the EU numbering index.

In some embodiments, the isolated antibody or/and the antigen-binding fragment thereof binding to Trop-2 binds to Trop-2 with a $K_D$ value of 1.00E-08 or less. In some embodiments, the antibody or/and the antigen-binding fragment thereof binds to human Trop-2. In other embodiments, the antibody or/and the antigen-binding fragment thereof binds to macaque Trop-2. In other embodiments, the antibody or/and the antigen-binding fragment thereof binds to human Trop-2 and cynomolgus monkey Trop-2. In some embodiments, the antibody or/and the antigen-binding fragment thereof has the following binding affinity ($K_D$) for human TROP-2: about 1.00E-08M or less, or about 1.16E-08 M or less. In some embodiments, the binding affinity $K_D$ of the antibody and the antigen-binding fragment thereof binding to TROP-2 provided herein is measured by Biacore.

In some embodiments, the antibody or/and the antigen-binding fragment thereof binding to TROP-2 has a binding $EC_{50}$ for human TROP-2 of about 0.035 nM to about 1.078 nM. In some embodiments, the antibody or/and the antigen-binding fragment thereof binding to TROP-2 has a binding $EC_{50}$ for human TROP-2 of about 1.078 nM or less, about 0.359 nM or less, or about 0.035 nM or less. In some embodiments, the $EC_{50}$ of the antibody and the antigen-binding fragment thereof binding to TROP-2 provided herein is measured by ELISA or FACS.

In some embodiments, the antibody or/and the antigen-binding fragment thereof binding to TROP-2 exhibits relatively strong internalization activity in cell lines expressing TROP-2 at varying amounts, and the amount of internalized antibody accumulates with increasing internalization time without exhibiting significant saturation at a relatively high concentration of antibody (20 nM).

In some embodiments, the antibody or/and the antigen-binding fragment thereof binding to TROP-2 exhibits the activity of inducing antibody-dependent cell-mediated cytotoxicity (ADCC) against cells expressing TROP-2 in different cell lines expressing TROP-2.

In some embodiments, the antibody or/and the antigen-binding fragment thereof binding to TROP-2 does not bind to tumor cells that do not express TROP-2.

In some embodiments, the isolated antibody binding to TROP-2 provided herein is a monoclonal antibody.

In some embodiments, the isolated antibody binding to TROP-2 provided herein is a monospecific antibody.

In some embodiments, the isolated antibody binding to TROP-2 provided herein is a multispecific antibody, e.g., a bispecific antibody, a trispecific antibody, or the like.

In some embodiments, the isolated antibody binding to TROP-2 provided herein is a fusion protein.

In one aspect, the present application provides an antibody-drug conjugate, which comprises the antibody or the antigen-binding fragment thereof binding to human TROP-2 provided by the present application linked to a therapeutic agent.

In one aspect, the present application provides an antibody-drug conjugate, which comprises the antibody or the antigen-binding fragment thereof binding to human TROP-2 provided by the present application linked to a therapeutic agent, wherein the therapeutic agent is a cytotoxic drug, a radioisotope, an immunomodulator, or an antibody. The cytotoxic drug may, e.g., be selected from the group consisting of alkaloids, antimetabolites, anti-tumor antibiotics, alkylating agents, platinum-based drugs, and the like; preferably, the cytotoxic drug is a microtubule inhibitor (including maytansinoid, auristatin, etc.) or a DNA-acting cytotoxic drug (including calicheamicin, duocarmycin, PBD (pyrrolobenzodiazepine), a topoisomerase I inhibitor, etc.).

In one aspect, the present application provides a pharmaceutical composition, which comprises the TROP-2 antibody or the antigen-binding fragment thereof provided herein and a pharmaceutically acceptable carrier.

In one aspect, the present application provides a pharmaceutical composition, which comprises a drug conjugate of the TROP-2 antibody or the antigen-binding fragment thereof provided herein and a pharmaceutically acceptable carrier.

In one aspect, the present application provides an isolated nucleic acid molecule encoding the antibody or the antigen-binding fragment thereof binding to human TROP-2 provided by the present application.

In one aspect, the present application provides an expression vector comprising the isolated nucleic acid molecule encoding the antibody or the antigen-binding fragment thereof binding to human TROP-2 provided by the present application.

In one aspect, the present application provides an expression vector comprising the isolated nucleic acid molecule encoding the antibody or the antigen-binding fragment thereof binding to human TROP-2 provided by the present application or a host cell having the isolated nucleic acid molecule described herein integrated into genome thereof.

In one aspect, the present application provides use of the antibody or the antigen-binding fragment thereof binding to human TROP-2 or the pharmaceutical composition thereof in preparing a medicament for the treatment of a TROP-2-mediated disease. In one aspect, the present application provides use of the antibody-drug conjugate of the present application in preparing a medicament for the treatment of a TROP-2-mediated disease.

In one aspect, the present application provides the antibody or the antigen-binding fragment thereof binding to human TROP-2 or the pharmaceutical composition thereof for use in the treatment of a TROP-2-mediated disease. In one aspect, the present application provides a method for treating a TROP-2-mediated disease, which comprises administering to a patient in need thereof a therapeutically effective amount of the antibody or the antigen-binding fragment thereof binding to human TROP-2 of the present application or the pharmaceutical composition comprising the antibody or the antigen-binding fragment thereof binding to human TROP-2 provided by the present application and a pharmaceutically acceptable carrier.

In one aspect, the TROP-2-mediated disease described above is cancer.

In one aspect, the present application provides a method for producing the antibody or the antigen-binding fragment thereof binding to human TROP-2, which comprises culturing the host cell under conditions enabling expression of the antibody or the antigen-binding fragment thereof and isolating the expressed antibody or antigen-binding fragment thereof.

The novel anti-TROP-2 antibody or the antigen-binding fragment thereof provided by the present application can bind to different epitopes of TROP-2, has high affinity and excellent internalization activity, shows strong cell killing activity and inhibitory activity against xenograft tumors in animals, has good safety, and is suitable for clinical application.

**Definitions and Explanations**

In the term "antibody or antigen-binding fragment thereof, antibody is used in its broadest sense and specifically encompasses intact monoclonal antibodies, polyclonal antibodies, multispecific antibodies (e.g., bispecific antibodies) formed by at least two intact antibodies, multifunctional antibodies, nanobodies, and fusion proteins, so long as they possess the desired biological activity. Examples of an antigen-binding fragment include, but are not limited to, a Fab fragment, a Fab' fragment, a F(ab')2 fragment, an Fd fragment, an Fv fragment, a dAb fragment, an isolated CDR region, a single-chain Fv molecule (scFv), and other antibody fragments.

The TROP-2 antibody or the antigen-binding fragment thereof of the present application may be of the IgG1, IgG2, IgG3, or IgG4 subtype. The TROP-2 antibody or the antigen-binding fragment thereof of the present application may be derived from any species, including but not limited to mouse, rat, rabbit, non-human primate (e.g., chimpanzee, cynomolgus monkey, spider monkey, or macaque), llama, and human. The TROP-2 antibody or the antigen-binding fragment thereof may be a murine antibody, a chimeric antibody, a humanized antibody, or a fully human antibody.

The term "Fab fragment" is composed of one light chain and the $C_H1$ and the variable regions of one heavy chain. The heavy chain of a Fab molecule is unable to form a disulfide bond with another heavy chain molecule.

The term "F(ab')$_2$ fragment" contains two light chains and two heavy chains comprising part of the constant region between the $C_H1$ and $C_H2$ domains, so that an interchain disulfide bond is formed between the two heavy chains. Thus, a F(ab')$_2$ fragment is composed of two Fab' fragments that are held together by a disulfide bond between the two heavy chains.

The term "Fv fragment" comprises the variable regions from both the heavy and light chains but lacks the constant regions.

The term "single-chain Fv molecule" (or "scFv" antibody) refers to an antibody fragment comprising the $V_H$ and $V_L$ domains of an antibody, where these domains are present in a single polypeptide chain. Generally, an Fv polypeptide further comprises a polypeptide linker between the $V_H$ and $V_L$ domains, and the linker enables the scFv to form the desired structure for antigen binding.

The term "CDR" (complementarity determining region) is also referred to as "hypervariable region". Natural four-chain antibodies typically comprise six CDRs, three in the heavy chain variable region and three in the light chain variable region.

The term "variable region": the antibody structural unit is composed of two pairs of polypeptide chains, each pair having one heavy chain and one light chain, and the N-terminal domain of each chain defining a region of about 100 to 110 or more amino acids primarily responsible for antigen recognition is the variable region.

The term "chimeric antibody" refers to antibodies in which a portion of the heavy and/or light chain is identical with or homologous to corresponding sequences of antibodies derived from a particular species or belonging to a particular antibody class or subclass, while the remainder of the chains is identical with or homologous to corresponding sequences of antibodies derived from another species or belonging to another antibody class or subclass, as well as fragments of such antibodies exhibiting the desired biological activity.

A "humanized antibody" is a chimeric antibody that contains minimal sequences derived from non-human immunoglobulin. The majority of a humanized antibody is a human immunoglobulin (receptor antibody), residues from a hypervariable region of which are replaced by residues from a hypervariable region of a non-human species (donor antibody) such as mouse, rat, rabbit, or non-human primate having the desired specificity, affinity, and capacity. In some instances, Fv framework region (FR) residues of the human immunoglobulin are replaced by corresponding non-human residues. Furthermore, a humanized antibody may comprise residues that are not found in the receptor antibody or the donor antibody. These modifications are made to further refine antibody performance. In general, a humanized antibody comprises substantially all of at least one, and typically two, variable domains, in which all or substantially all of the hypervariable regions correspond to those of a non-human immunoglobulin and all or substantially all of the FR regions are those of a human immunoglobulin sequence. A humanized antibody optionally also comprises at least a portion of an immunoglobulin (typically a human immunoglobulin) constant region.

The anti-TROP-2 antibody disclosed herein that has one or more amino acid substitutions, insertions, deletions, or combinations thereof in the CDRs or variable regions retains the biological activity of the corresponding anti-TROP-2 antibody without amino acid substitutions, insertions, or deletions. Thus, the anti-TROP-2 antibody variants provided herein retain binding to TROP-2. In some embodiments, variants of the CDR or variable region amino acid sequences are provided, wherein the variants comprise 1, 2, 3, 4, 5, 6, 7, 8, 9, 10, or more amino acid substitutions, insertions, deletions, or combinations thereof. In another embodiment, the amino acid substitution is a conservative substitution.

The term "identity" is also known as homology. The percent identity between two sequences is a function of identical positions shared by the sequences (i.e., %identity = number of identical positions/total number of positions $\times$ 100), where the number of gaps that need to be introduced to produce an optimal alignment of the two sequences and the length of each gap should be taken into consideration. As shown in the following non-limiting examples, comparison of sequences and determination of percent identity between two sequences can be accomplished using a mathematical algorithm. The percent identity between two amino acid sequences can be determined using the algorithm of E. Meyers and W. Miller (Comput. Appl. Biosci., 4:11-17 (1988)), which has been incorporated into the ALIGN program (version 2.0) and uses a PAM120 residue weight table with a gap length penalty of 12 and a gap penalty of 4. In addition, the percent identity of two amino acid sequences can be determined using the algorithm of Needleman and Wunsch (J. Mol. Biol. 484-453 (1970)), which has been incorporated into the GAP program in the GCG software package (available at www.gcg.com) and uses the Blossum 62 matrix or the PAM250 matrix with a gap weight of 16, 14, 12, 10, 8, 6, or 4 and a length weight of 1, 2, 3, 4, 5, or 6.

As used herein, the term "derived", when used to refer to a molecule or polypeptide relative to a reference antibody or other binding proteins, means a molecule or polypeptide that can specifically bind to the same epitope as the reference antibody or other binding proteins.

The term "isolated" means that a target compound (e.g., an antibody or a nucleic acid) has been isolated from its natural environment.

As used herein, the term "$EC_{50}$" refers to a concentration of an antibody that induces 50% of the maximal effective response. As used herein, the term "$IC_{50}$" refers to a concentration of an antibody that induces 50% of the maximal inhibitory response. Both $EC_{50}$ and $IC_{50}$ may be measured by ELISA or FACS assay or any other methods known in the art.

The term "$K_D$" as used herein refers to the dissociation constant, expressed in molar concentration (M). The $K_D$ value of an antibody can be determined using methods well known in the art. One preferred method for determining the $K_D$ of an antibody is to use surface plasmon resonance, more preferably a biosensor system, such as the Biacore system.

The term "cancer" refers to a physiological condition in mammals that is typically characterized by unregulated cell growth.

The term "treating" or "treatment" means administering the compound or formulation described herein to prevent, ameliorate, or eliminate a disease or one or more symptoms associated with the disease, including: (i) preventing the occurrence of a disease or disease state in a mammal, particularly when such a mammal is predisposed to the disease state but has not yet been diagnosed with it; (ii) inhibiting a disease or disease state, i.e., arresting its progression; (iii) alleviating a disease or disease state, i.e., causing its regression; and (iv) reducing any direct or indirect pathological consequences of a disease or disease state.

The term "therapeutically effective amount" refers to an amount of the compound of the present application for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but can be determined routinely by those skilled in the art in accordance with their knowledge and the present disclosure. The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "solvate" refers to a substance formed by association of a compound with a solvent molecule.

The term "pharmaceutical composition" refers to a mixture consisting of one or more of the compounds or the salts thereof of the present application and a pharmaceutically acceptable carrier. The pharmaceutical composition is intended to facilitate the administration of the compound of the present application to an organism .

The term "pharmaceutically acceptable carrier" refers to those excipients that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present application is a human. The terms "patient" and "subject" are used interchangeably unless otherwise indicated.

Unless otherwise specified clearly herein, singular terms encompass plural referents, and vice versa. Similarly, unless otherwise specified clearly herein, the word "or" is intended to include "and".

As used herein, unless otherwise stated, the terms "comprise", "comprises" and "comprising" or equivalents thereof (contain, contains, containing, include, includes, and including) are open-ended statements and mean that elements, components and steps that are not specified may also be included in addition to those listed.

As used herein, unless otherwise stated, all numbers expressing the amounts of ingredients, measurements, or reaction conditions used herein are to be understood as being modified in all instances by the term "about". As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to ±5%, for example, fluctuating within a particular numerical range given ±2%, ±1%, or ±0.5%. Where a particular value is given in the present application or in the claims, unless otherwise stated, "about" should be considered to mean being within an acceptable error range for that particular value. In this context, unless otherwise stated, the values of the parameters or conditions in a step are all modified by "about" by default.

## BRIEF DESCRIPTION OF THE DRAWINGS

FIG. 1 shows the binding of humanized antibodies of Ab2, chimeric antibody and humanized antibodies of Ab21, and chimeric antibody and humanized antibodies of Ab35 to human Trop-2-His at protein level as detected by ELISA (enzyme linked immunosorbent assay).

FIG. 2 shows the binding of humanized antibodies of Ab2, humanized antibodies of Ab21, and humanized antibodies of Ab35 to A549 lung tumor cells as detected by flow cytometry.

FIG. 3 shows the internalization of humanized antibodies of Ab2 and humanized antibodies of Ab21 in HCC38 cells (culturing for 2 h) as detected by flow cytometry.

FIG. 4 shows the internalization of humanized antibodies of Ab35 in OVCAR3 cells (culturing for 2 h) as detected by flow cytometry.

FIG. 5 shows the internalization of humanized antibodies hzAb2-2.4, hzAb21-2.1, and hz-Ab35-2.3 in HCC38 cells (culturing for 2 h, 4 h, or 6 h) as detected by flow cytometry.

FIG. 6 shows the internalization of the positive control datopotamab in HCC38 cells (culturing for 2 h, 4 h, or 6 h) as detected by flow cytometry.

FIG. 7 shows the ADCC activity of chimeric antibody and humanized antibodies of Ab2, chimeric antibody and humanized antibodies of Ab21, and chimeric antibody and humanized antibodies of Ab35 as detected based on reporter gene assay, wherein the effector cell is Jurkat-CD16a v158-NFAT-luciferase and the target cell is NCI-H322 or HCC1806 cell.

FIG. 8 shows the ADCC activity of chimeric antibody and humanized antibodies of Ab2, chimeric antibody and humanized antibodies of Ab21, and chimeric antibody and humanized antibodies of Ab35 as detected based on killing effect of PBMC, wherein the effector cell is PBMC and the target cell is NCI-H322 or HCC1806 cell.

## DETAILED DESCRIPTION

Although the foregoing invention has been described in considerable detail by providing illustrations and examples for the purpose of clear understanding, it will be apparent to those of ordinary skills in the art in light of the teachings of the present application that certain changes and modifications can be made to the present application without departing from the spirit and scope of the appended claims. The following examples are provided for illustrative purposes only and are not intended to be limiting. Those skilled in the art will readily identify a variety of noncritical parameters that may be changed or modified to produce substantially similar results.

The positive control drug TINA (also called datopotamab) used in the examples of the present application is the TROP-2 antibody developed by Daiichi Sankyo Co., Ltd., the heavy chain sequence of the positive control drug corresponds to serial number 12 in CN105849126A, and the light chain sequence of the positive control drug corresponds to serial number 18 in CN105849126A. The positive control drug is prepared according to the conventional method for antibodies; vector construction is first carried out, followed by transfection into eukaryotic cells, purification, and expression.

Unless otherwise stated, the practice of the present application uses techniques in the conventional molecular biology, cell biology, biochemistry, and immunology well known in the art and described in the following: e.g., Methods in Molecular Biology, Humana Press; Molecular Cloning: A Laboratory Manual, 2nd Edition (Sambrook et al., 1989); Current Protocols in Immunology (Eds. J. E. Coligan et al., 1991); Immunobiology (C. A. Janeway and P. Travers, 1997); Antibodies (P. Finch, 1997); Antibodies: a practical approach (Ed. D. Catty, IRL Press, 1988-1989); Monoclonal antibodies: a practical approach (Eds. P. Shepherd and C. Dean, Oxford University Press, 2000); Phage display: a laboratory manual (C. Barbas III et al., Cold Spring Harbor Laboratory Press, 2001); and Using antibodies: a laboratory manual (E. Harlow and D. Lane, Cold Spring Harbor Laboratory Press, 1999).

*Immunoconjugate*

In some embodiments, the present application provides an immunoconjugate of the antibody or the antigen-binding fragment thereof binding to TROP-2. In some embodiments, the immunoconjugate is an antibody-drug conjugate comprising the antibody or the antigen-binding fragment thereof binding to human TROP-2 provided by the present application linked to or conjugated to a therapeutic agent. A therapeutic agent that may be linked to or conjugated to the antibody or the antigen-binding fragment thereof binding to TROP-2 may include, but is not limited to, a cytotoxic drug, a radioisotope, an immunomodulator, or an antibody. In some embodiments, the antibody or the antigen-binding fragment thereof binding to TROP-2 is conjugated to the therapeutic agent directly. In some embodiments, the antibody or the antigen-binding fragment thereof binding to TROP-2 is conjugated to the therapeutic agent via a linker.

*Chimeric antigen receptor (CAR)*

The present application further provides a chimeric antigen receptor comprising the antibody or the antigen-binding fragment thereof binding to human TROP-2 of the present application.

CARs binding to human TROP-2 may comprise: (a) an extracellular antigen-binding domain comprising an antibody or an antigen-binding fragment thereof binding to human TROP-2; (b) a transmembrane domain; and (c) an intracellular signaling domain.

Elements that enhance T cell expansion, persistence, and anti-tumor activity, such as cytokines and co-stimulatory ligands, may further be added to CARs.

The present application further provides an engineered immune effector cell comprising the CAR provided by the present application.

*Pharmaceutical composition*

The present application provides a pharmaceutical composition comprising an isolated antibody or an antigen-binding fragment thereof binding to TROP-2, or a nucleic acid encoding the antibody or fragment, or the immunoconjugate described herein, and further one or more pharmaceutically acceptable carriers. In some embodiments, the pharmaceutical composition comprises any monoclonal antibody in the chimeric antibodies and humanized antibodies described herein and a pharmaceutically acceptable carrier. The pharmaceutically acceptable carrier includes, for example, stabilizers, excipients, diluents, encapsulating materials, fillers, buffering agents, vehicles, or other agents.

*Hybridoma*

The antibody or the antigen-binding fragment thereof binding to Trop-2 provided by the present application is produced by hybridomas. Accordingly, the present application also includes any hybridoma that produces the antibody or the antigen-binding fragment thereof disclosed herein.

*Isolated nucleic acid*

The present application provides an isolated nucleic acid comprising a sequence encoding the antibody or the antigen-binding fragment thereof binding to TROP-2 described herein. In some embodiments, the nucleic acid may encode an amino acid sequence comprising a heavy chain variable region or/and a light chain variable region of the antibody or the antigen-binding fragment thereof described herein. In other embodiments, the nucleic acid may encode an amino acid sequence comprising a heavy chain or/and a light chain of the antibody described herein. Nucleotide sequences of some of the variable regions and the heavy and light chains of the antibody are illustratively listed in the sequence listing.

*Vector*

The present application provides a vector comprising the isolated nucleic acid. In some embodiments, the vector is a cloning vector; in other embodiments, the vector is an expression vector.

The expression vector is optionally any expression vector capable of expressing the antibody or the antigen-binding fragment thereof described herein.

*Host cell*

In some embodiments, the present application provides a host cell comprising the vector, the host cell being a suitable host cell for cloning or encoding the antibody or the antigen-binding fragment thereof binding to TROP-2. In some embodiments, the host cell is a prokaryotic cell. In other embodiments, the host cell is a eukaryotic cell. In some embodiments, the host cell is selected from the group consisting of a yeast cell, a mammalian cell, and other cells suitable for preparing an antibody or an antigen-binding fragment thereof. Mammalian cells are, for example, Chinese hamster ovary (CHO) cells.

*Method for preparing antibody or antigen-binding fragment thereof binding to TROP-2*

In some embodiments, the present application provides a method for preparing an isolated antibody or an antigen-binding fragment thereof binding to TROP-2, which comprises culturing a host cell comprising a nucleic acid encoding the antibody or the antigen-binding fragment thereof under conditions suitable for expression of the antibody or the antigen-binding fragment thereof and isolating the antibody or the antigen-binding fragment thereof from the host cell or host cell culture medium. To produce the antibody or the antigen-binding fragment thereof binding to TROP-2, the nucleic acid encoding the antibody or the antigen-binding fragment thereof is isolated and inserted into one or more vectors for further cloning or/and expression in a host cell. The nucleic acid can be obtained using various methods known in the art, such as gene cloning, gene splicing, and chemical synthesis.

*Use/treatment method*

The present application provides use of the isolated antibody or the antigen-binding fragment thereof binding to TROP-2 or a method of using the antibody or the antigen-binding fragment thereof for cancer treatment; in some embodiments, administering to a subject a therapeutically effective amount of the isolated antibody or the antigen-binding fragment thereof binding to TROP-2 described herein can reduce or inhibit tumor cell growth. In some embodiments, administering to a subject a therapeutically effective amount of the isolated antibody or the antigen-binding fragment thereof binding to TROP-2 described herein can treat cancer.

The present application provides use of the pharmaceutical composition of the present application or a method of using the pharmaceutical composition for cancer treatment; in some embodiments, administering to a subject a therapeutically effective amount of the pharmaceutical composition of the present application can reduce or inhibit tumor cell growth. In some embodiments, administering to a subject a therapeutically effective amount of the pharmaceutical composition of the present application can treat cancer.

The present application provides use of the antibody-drug conjugate of the present application or a method of using the antibody-drug conjugate for cancer treatment; in some embodiments, administering to a subject a therapeutically effective amount of the antibody-drug conjugate of the present application can reduce or inhibit tumor cell growth. In some embodiments, administering to a subject a therapeutically effective amount of the antibody-drug conjugate of the present application can treat cancer.

Subjects in need of treatment include those with a disease or condition, as well as those who may have the disease or condition and whose purpose is to prevent, delay, or alleviate the disease or condition. As used herein, "cancer" refers to a physiological condition in mammals that is typically characterized by unregulated cell growth. Examples of cancer include, but are not limited to, leukemia, lymphoma, ovarian cancer, breast cancer, endometrial cancer, colon cancer, colorectal cancer, rectal cancer, bladder cancer, urothelial cancer, lung cancer, bronchial cancer, bone cancer, prostate cancer, pancreatic cancer, gastric cancer, hepatocellular carcinoma, gallbladder cancer, bile duct cancer, esophageal cancer, renal cell carcinoma, thyroid cancer, head and neck cancer, testicular cancer, endocrine adenocarcinoma, adrenal carcinoma, pituitary gland cancer, skin cancer, soft tissue cancer, vascular cancer, brain cancer, nerve cancer, eye cancer, meningeal cancer, oropharyngeal cancer, hypopharynx cancer, cervical cancer, uterine cancer, glioblastoma, medulloblastoma, astrocytoma, glioma, meningioma, gastrinoma, neuroblastoma, melanoma, acute myeloid leukemia, myelodysplastic syndrome, and sarcoma.

**Example 1. Construction of Cell Srain with High Trop-2 Expression**

The DNA sequence of the full-length open reading frame of human Trop-2 was synthesized by General Biosystems (Anhui) Co., Ltd. and then subcloned into the expression vector pcDNA3.1 (Invitrogen, Cat. No.: V-79020), which was transfected into blank CHO-K1 cells using the Lipofectamine3000 (Gibco) transfection reagent. The cells were subjected to screening for 2-3 weeks with geneticin, then the limiting dilution method was performed to separate and obtain single clones. The expression amount of Trop-2 on the cell surfaces of the monoclonal CHO-K1 cell strains obtained by subcloning with the limiting dilution method was detected by FACS to select CHO-K1/hTrop-2 monoclonal cell strains with a high expression amount of Trop-2. The full-length amino acid sequence of the human Trop-2 is set forth in SEQ ID NO: 1.

**Example 2. Production of Murine Trop-2 Monoclonal Antibodies**

Obtaining mouse anti-human Trop-2 antibodies: mice were immunized with human Trop-2-hFc. Specifically, the extracellular domain of human Trop-2 was recombinantly linked to human IgG Fc to form TROP2-hFc (SEQ ID NO: 2). The DNA sequences were synthesized by General Biosystems (Anhui) Co., Ltd., and each was subcloned into the expression vector pcDNA3.1 (Invitrogen, Cat. No.: V-79020). After transient expression in FreeStyle293 cells, human Trop-2-hFc was purified using a protein A column (GE Healthcare). BALB/c and A/J mice (Shanghai Sippe-Bk Lab Animal Co., Ltd.) were immunized subcutaneously every 2 weeks for 6 weeks with the recombinant human Trop-2-hFc protein (100 $\mu$g/mouse) emulsified with an equal volume of complete/incomplete Freund's adjuvant. Three days before fusion, the mice were injected intravenously with an antigen without an adjuvant to boost the immunization. Spleen cells ($1 \times 10^8$) from the immunized mice were fused with SP2/0 myeloma cells ($1 \times 10^7$) using the electrofusion system (BTX, 2001). After fusion, the cells were added to a 96-well plate at 0.1 mL/well and incubated in an incubator at 37°C with 5% $CO_2$. On day 1, the cells were fed by additionally adding 0.1 mL of HAT medium containing serum and 2× methotrexate to each well. On days 3 and 7, 0.1 mL of the medium from each well was replaced with 0.1 mL of fresh HT medium. Screening was typically performed between days 9-14, and hybridoma culture supernatants reacting with human Trop-2-His (SEQ ID NO: 3) were selected by ELISA.

**Example 3: cDNA Sequence Cloning of Anti-Trop-2 Antibodies**

Total RNA isolated from a hybridoma cell line producing a human Trop-2 antibody by using the RNeasy mini kit (Qiagen, Cat. No.: 74104) was used as the template. The first strand cDNA was then synthesized according to the manufacturer's instructions for the cDNA synthesis kit, the PrimeScript1st strand cDNA Synthesis kit (TAKARA, Cat. No.: D6110A). The cDNA product was then subjected to PCR in 50 $\mu$L of a reaction mixture using a degenerate mouse IgG primer (Kettleborough CA et al., European Journal of Immunology, 23:206-211(1993), Strebe N et al., Antibody Engineering, 1:3-14 (2010)). The reaction was carried out in an S1000TM thermal cycler (Bio-Rad, Cat. No.: 184-2000), and the following cycles were carried out 25 times: denaturation at 94°C for 1.5 min, annealing at 50°C for 1 min, and extension at 72°C for 1 min. At the end of the 25th cycle, the reaction mixture was incubated at 72°C for 5 min to allow for a complete reaction. The PCR mixture was subjected to electrophoresis in a 1% agarose/Tris-borate gel containing 0.5 $\mu$g/mL ethidium bromide. A DNA fragment of the expected size was cut off from the gel and purified. 3 $\mu$L of the purified PCR product was cloned into the pMD-18T vector (Takara, Cat. No.: D101A), which was then transformed into the OneTOP10 chemically competent Escherichia coli (Invitrogen, Cat. No.: C4040-03). Clones were screened by colony PCR using the universal M13 forward and reverse primers, and 6 positive clones were selected from each reaction for DNA sequencing in both directions using M13 forward primers and M13 reverse primers.

The heavy chain variable region sequences and light chain variable region sequences of antibodies were amplified from the corresponding hybridoma clones, and the antibodies included Ab2, Ab21, and Ab35. These antibodies showed desirable properties, such as binding to tumor cells expressing human Trop-2, species cross-binding ability, and relatively high internalization rate.

Heavy chain variable region of Ab2 (SEQ ID NO: 23):

EVQLVESGPGLVAPSQSLSITCTVS<u>GFSLTYYGID</u>WVRQPPGKGLEWLG<u>VIWGSGNTKYNSALMS</u>RLSISK DNSKSQVFLRMNSLQSDDTAIYYCAR<u>FYEGFAY</u>WGQGTLVTVSA

Light chain variable region of Ab2 (SEQ ID NO: 24):

AIVLTQSPSSMYASLGERVTITC<u>KASQDINSYLS</u>WFQQKPGKSPKTLIY<u>RANRLVD</u>GVPSRFSGSGSGQDY SLTISSLEYEDMGIYYC<u>LQYDEFPLT</u>FGVGTKLELK

Table 1. CDR sequences of Ab2

| Name | Sequence | No. | Name | Sequence | No. |
|------|----------|-----|------|----------|-----|
| HCDR1 | GFSLTYYGID | SEQ ID NO:5 | LCDR1 | KASQDINSYLS | SEQ ID NO:8 |
| HCDR2 | VIWGSGNTKYNSALMS | SEQ ID NO:6 | LCDR2 | RANRLVD | SEQ ID NO:9 |
| HCDR3 | FYEGFAY | SEQ ID NO:7 | LCDR3 | LQYDEFPLT | SEQ ID NO:10 |

Heavy chain variable region of Ab21 (SEQ ID NO: 25):

EVQLKQSGPGLVQPSQSLSITCTVSGFSLRNYGVHWVRQSPGKGLEWLGVIWSSGTTDYNAAFISRLSITK
DNSKSQVFFKMNSLQVDDTAIYNCARDGDYDYYTMDYWGQGTSVTVSS

Light chain variable region of Ab21 (SEQ ID NO:26:

AIVLTQSPASLAVSLGQRATISCRASKSVSTSAYSYIHWYQQKPGQPPKLLIYLTSNLESGVPARFSGSGSGT
DFTLNIHPVEEEDAATYYCQHSRELPYTFGGGTKLEIK

Table 2. CDR sequences of Ab21

| Name | Sequence | No. | Name | Sequence | No. |
|------|----------|-----|------|----------|-----|
| HCDR1 | GFSLRNYGVH | SEQ ID NO:11 | LCDR1 | RASKSVSTSAYSYIH | SEQ ID NO:14 |
| HCDR2 | VIWSSGTTDYNAAFISR | SEQ ID NO:12 | LCDR2 | LTSNLES | SEQ ID NO:15 |
| HCDR3 | DGDYDYYTMDY | SEQ ID NO:13 | LCDR3 | QHSRELPYT | SEQ ID NO:16 |

Heavy chain variable region of Ab35 (SEQ ID NO: 27):

EVQLQQSGPDLVRPGASVKLSCKASGYTFTSSDINWVKQRPGQGLEWIGWIYPRDGSKKYNEKFKGKAT
LTVDTSSSTAYMEVHSLTSEDSAVYFCATRFATVVSNYGLDPWGQGTSVTVSS

Light chain variable region of Ab35 (SEQ ID NO: 28):

AIVLTQSPAIMSASPGEKVTMTCSASSSVNYIYWYQRKPGSSPRLLIYDTSNLASGVPVRFSGSGSGTSYSL
TISRMEAEDAATYYCQQWNSYPLTFGAGTKLELK

Table 3. CDR sequences of Ab35

| Name | Sequence | No. | Name | Sequence | No. |
|------|----------|-----|------|----------|-----|
| HCDR1 | GYTFTSSDIN | SEQ ID NO:17 | LCDR1 | SASSSVNYIY | SEQ ID NO:20 |
| HCDR2 | WIYPRDGSKKYNEKFKG | SEQ ID NO:18 | LCDR2 | DTSNLAS | SEQ ID NO:21 |
| HCDR3 | RFATVVSNYGLDP | SEQ ID NO:19 | LCDR3 | QQWNSYPLT | SEQ ID NO:22 |

**Example 4. Construction and Expression of Chimeric Antibodies**

cDNAs of the mouse VL regions obtained by PCR cloning were each linked to a human κ chain constant region (amino acid sequence set forth in SEQ ID NO: 30) to construct chimeric light chains of ch-Ab2, ch-Ab21, and ch-Ab35 (amino acid sequences set forth in SEQ ID NO: 74, SEQ ID NO: 76, and SEQ ID NO:78, respectively). cDNAs of the mouse VH regions obtained by PCR cloning were each linked to a human IgG1 constant region (amino acid sequence set forth in SEQ ID NO: 29) to construct chimeric heavy chains of ch-Ab2, ch-Ab21, and ch-Ab35 (amino acid sequences set forth in SEQ ID NO: 73, SEQ ID NO: 75, and SEQ ID NO: 77, respectively). The 5' end of the mouse cDNA sequence was modified using a PCR primer designed to add leader sequences to both the light and heavy chains.

ExpiCHO cells (100 mL, $6.3 \times 10^6$ cells/mL) were transfected with 40 μg of each chimeric heavy chain expression plasmid and each light chain expression plasmid and cultured for 10-12 days. The chimeric antibodies in the supernatant were then purified using a protein A column (GE Healthcare). The binding of the chimeric antibodies to Trop-2 was measured by ELISA and Biacore, and the results indicated that the chimeric antibodies bound to Trop-2 with an affinity comparable to that of the murine antibodies.

## Example 5. Humanization Design of Antibodies

Antibodies Ab2, Ab21, and Ab35 were humanized using the CDR grafting method (see, e.g., U.S. Pat. No. 5,225,539). Murine antibodies Ab2, Ab21, and Ab35 were subjected to homology modeling by using MOE (molecular operating environment), resulting in protein structural models of the Fv domains. The VH and VL amino acid sequences of the murine antibodies Ab2, Ab21, and Ab35 were input into the international ImMunoGeneTics information system-related tool website (http://www.imgt.org/3Dstructure-DB/cgi/DomainGapAlign.cgi), from which human germline antibody sequences highly identical to mouse antibodies Ab2, Ab21, and Ab35 were selected. Complementarity determining regions (CDRs) in the VHs and VLs of murine antibodies Ab2, Ab21, and Ab35 were grafted to the template human antibodies. The CDR boundaries of an antibody can be named or identified using the Kabat, Chothia, IMGT, or Al-Lazikani scheme (Al-Lazikani, B., Chothia, C., Lesk, A. M., J. Mol. Biol., 273(4), 927 (1997); Chothia, C. et al., J Mol Biol, Dec 5; 186(3):651-63 (1985); Chothia, C. and Lesk, A. M., J. Mol. Biol., 196, 901(1987); Chothia, C. et al., Nature, Dec 21-28; 342(6252):877-83 (1989); Kabat E. A. et al.). For Ab2, the selected template human VH was a combination of IGHV3-53*01, IGHV4-4*08, and IGHJ4*01, and the selected template human VL was a combination of IGKV1-39*01 and 1GKJ1*01. For Ab21, the selected template human VH was a combination of IGHV3-15*01 and IGHJ4*01, and the selected template human VL was a combination of IGKV3-11*01 and 1GKJ1*01. For Ab35, the selected template human VH was a combination of IGHV1-46*01 and IGHJ4*01, and the selected template human VL was a combination of IGKV3-11*01 and 1GKJ1*01.

The amino acid sequences of the CDRs of the template human antibodies were replaced with the CDRs of the mouse antibodies. The essential amino acid sequences from the VHs and VLs of the mouse antibodies Ab2, Ab21, and Ab35 were grafted to the VH and VL frameworks of the above template human germline antibodies to obtain functional humanized antibodies. For the VHs and VLs of antibodies Ab2, Ab21, and Ab35, several sites of the framework amino acids of the above template human antibodies were back mutated to the corresponding amino acid sequences in the mouse antibodies Ab2, Ab21, and Ab35.

Table 4. Sequence numbers of humanized antibodies

| Antibody | Heavy chain CDR1-3 | Light chain CDR1-3 | Heavy chain variable region | Light chain variable region | Heavy chain | Light chain |
|---|---|---|---|---|---|---|
| hz-Ab2-1.1 | SEQ ID NO:5, 31, 7 | SEQ ID NO:8, 34, 10 | SEQ ID NO:36 | SEQ ID NO: 40 | SEQ ID NO: 42 | SEQ ID NO:46 |
| hz-Ab2-1.2 | SEQ ID NO:5, 32, 7 | | SEQ ID NO:37 | | SEQ ID NO: 43 | |
| hz-Ab2-1.3 | SEQ ID NO:5, 31, 7 | | SEQ ID NO:38 | | SEQ ID NO: 44 | |
| hz-Ab2-1.4 | SEQ ID NO:5, 31, 7 | | SEQ ID NO:39 | | SEQ ID NO: 45 | |

(continued)

| Antibody | Heavy chain CDR1-3 | Light chain CDR1-3 | Heavy chain variable region | Light chain variable region | Heavy chain | Light chain |
|---|---|---|---|---|---|---|
| hz-Ab2-2.1 | SEQ ID NO:5, 31, 7 | SEQ ID NO: 33, 34, 35 | SEQ ID NO:36 | SEQ ID NO: 41 | SEQ ID NO: 42 | SEQ ID NO:47 |
| hz-Ab2-2.2 | SEQ ID NO:5, 32, 7 | | SEQ ID NO:37 | | SEQ ID NO: 43 | |
| hz-Ab2-2.3 | SEQ ID NO:5, 31, 7 | | SEQ ID NO:38 | | SEQ ID NO: 44 | |
| hz-Ab2-2.4 | SEQ ID NO:5, 31, 7 | | SEQ ID NO:39 | | SEQ ID NO: 45 | |
| hz-Ab21-1.1 | SEQ ID NO: 48, 49, 13 | SEQ ID NO: 14, 15, 16 | SEQ ID NO:50 | SEQ ID NO: 54 | SEQ ID NO: 56 | SEQ ID NO:60 |
| hz-Ab21-1.2 | SEQ ID NO: 48, 12, 13 | | SEQ ID NO:51 | | SEQ ID NO: 57 | |
| hz-Ab21-1.3 | SEQ ID NO: 48, 49, 13 | | SEQ ID NO:52 | | SEQ ID NO: 58 | |
| hz-Ab21-1.4 | SEQ ID NO: 48, 12, 13 | | SEQ ID NO:53 | | SEQ ID NO: 59 | |
| hz-Ab21-2.1 | SEQ ID NO: 48, 49, 13 | SEQ ID NO: 14, 15, 16 | SEQ ID NO:50 | SEQ ID NO: 55 | SEQ ID NO: 56 | SEQ ID NO:61 |
| hz-Ab21-2.2 | SEQ ID NO: 48, 12, 13 | | SEQ ID NO:51 | | SEQ ID NO: 57 | |
| hz-Ab21-2.3 | SEQ ID NO: 48, 49, 13 | | SEQ ID NO:52 | | SEQ ID NO: 58 | |
| hz-Ab21-2.4 | SEQ ID NO: 48, 12, 13 | | SEQ ID NO:53 | | SEQ ID NO: 59 | |
| hz-Ab35-1.1 | SEQ ID NO: 17, 62, 19 | SEQ ID NO: 20, 21, 22 | SEQ ID NO:63 | SEQ ID NO: 66 | SEQ ID NO: 68 | SEQ ID NO:71 |
| hz-Ab35-1.2 | SEQ ID NO: 17, 18, 19 | | SEQ ID NO:64 | | SEQ ID NO: 69 | |
| hz-Ab35-1.3 | SEQ ID NO: 17, 18, 19 | | SEQ ID NO:65 | | SEQ ID NO: 70 | |
| hz-Ab35-2.1 | SEQ ID NO: 17, 62, 19 | SEQ ID NO: 20, 21, 22 | SEQ ID NO:63 | SEQ ID NO: 67 | SEQ ID NO: 68 | SEQ ID NO: 72 |
| hz-Ab35-2.2 | SEQ ID NO: 17, 18, 19 | | SEQ ID NO:64 | | SEQ ID NO: 69 | |
| hz-Ab35-2.3 | SEQ ID NO: 17, 18, 19 | | SEQ ID NO:65 | | SEQ ID NO: 70 | |

## Example 6. Antibody Binding Assay at Protein Level

Human Trop-2-His (SEQ ID NO: 3) protein was diluted to 2 $\mu$g/mL with PBS (SmartBuffer, PBS-2050) buffer at pH 7.4, added to a 96-well microplate at 100 $\mu$L/well, and incubated overnight at 4°C. After the liquid was discarded, 2% BSA (SIGMA, B2064-100G) diluted with PBS was added at 300 $\mu$L/well for blocking, and the mixture was incubated at 37°C for 2 h. After the blocking was completed, the blocking buffer was discarded, the plate was washed 3 times with PBST buffer (pH 7.4 PBS containing 0.1% tween-20), the antibody solution diluted in a gradient was added at 100

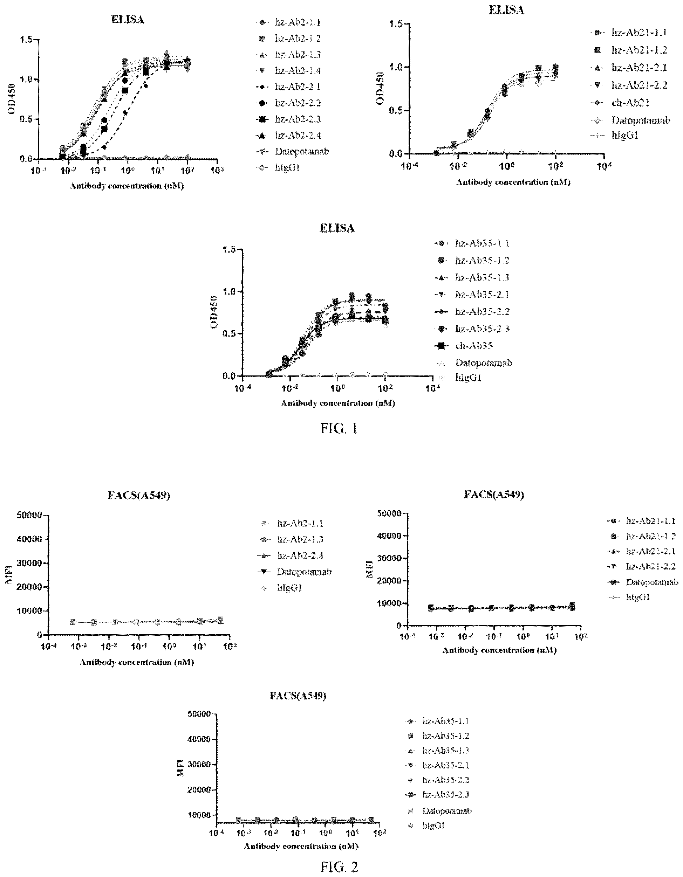

μL/well, and the mixture was incubated at 37°C for 1 h. After the incubation was completed, the plate was washed 3 times with PBST, the goat anti-human IgG (H+L) (JacksonImmunoResearch, 109-035-098, 1:5000 dilution) was added at 100 μL/well, and the mixture was incubated at 37°C for 1 h. After the plate was washed 3 times with PBST, a TMB chromogenic substrate (TIANGEN, PA107-01) was added at 100 μL/well. The mixture was incubated at room temperature for 10-15 min, and then 1 M $H_2SO_4$ was added at 50 μL/well to terminate the reaction. The absorbance values at 450 nm were taken using a microplate reader. The binding curves of antibodies for antigen were fitted using software, and the $EC_{50}$ values were calculated. The binding activity of the antibodies for the proteins is shown in Table 5-1, Table 5-2, Table 5-3, and FIG. 1. The results show that the binding activity of the humanized antibodies for human Trop-2-His protein was comparable to that of the parental chimeric antibodies.

Table 5-1. Affinity $EC_{50}$ of chimeric antibody and humanized antibodies of Ab2 for Trop-2-His protein (nM)

| Antibody | Affinity, $EC_{50}$ (nM) |
|---|---|
| ch-Ab2 | 0.085 |
| hz-Ab2-1.1 | 0.082 |
| hz-Ab2-1.2 | 0.076 |
| hz-Ab2-1.3 | 0.081 |
| hz-Ab2-1.4 | 0.068 |
| hz-Ab2-2.1 | 1.078 |
| hz-Ab2-2.2 | 0.218 |
| hz-Ab2-2.3 | 0.359 |
| hz-Ab2-2.4 | 0.100 |
| TINA | 0.083 |

Table 5-2. Affinity $EC_{50}$ of chimeric antibody and humanized antibodies of Ab21 for Trop-2-His protein (nM)

| Antibody | Affinity, $EC_{50}$ (nM) |
|---|---|
| ch-Ab21 | 0.165 |
| hz-Ab21-1.1 | 0.162 |
| hz-Ab21-1.2 | 0.248 |
| hz-Ab21-2.1 | 0.214 |
| hz-Ab21-2.2 | 0.223 |
| TINA | 0.166 |

Table 5-3. Affinity $EC_{50}$ of chimeric antibody and humanized antibodies of Ab35 for Trop-2-His protein (nM)

| Antibody | Affinity, $EC_{50}$ (nM) |
|---|---|
| ch-Ab35 | 0.043 |
| hz-Ab35-1.1 | 0.052 |
| hz-Ab35-1.2 | 0.035 |
| hz-Ab35-1.3 | 0.042 |
| hz-Ab35-2.1 | 0.055 |
| hz-Ab35-2.2 | 0.054 |
| hz-Ab35-2.3 | 0.065 |
| TINA | 0.083 |

## Example 7. Trop-2 Expression Levels on Different Cells

Cells were prepared into a cell suspension ($0.5 \times 10^6$-$1 \times 10^6$ cells/mL) using FACS buffer (Miltenyi Biotec, 130-091-221), and the cell suspension was added to a 96-well V-bottom plate at 100 μL/well. After centrifugation and removal of the supernatant, TINA and human IgG1 control antibody each diluted to 3 μg/mL with the FACS buffer was added at 50 μL/well separately, and the mixture was incubated in the dark in a 4°C refrigerator for 1 h. The plate was then washed 3 times with the FACS buffer by centrifugation at 300 g, and Alexa Fluor 488 goat anti-human IgG (H+L) (Bio-legend, 409304, 1:500 dilution) at working concentration was added. The mixture was incubated in the dark in a 4°C refrigerator for 40 min. The plate was then washed 3 times with the FACS buffer by centrifugation at 300 g, and the geometric mean fluorescence intensity (MFI) was measured on a Sartorius iQue3 flow cytometer. According to the ratio of the MFI value detected for TINA to the value detected for the IgG1 control antibody, the expression amount of Trop-2 on the cell surfaces of NCI-H322, HCC38, and HCC1806 was considered to be high, the expression of Trop-2 on the cell surfaces of Capan-1, PC-3, HUVEC, and OVCAR-3 was considered to be medium, and Trop-2 was not expressed on the cell surface of A549.

Table 6. Trop-2 expression on different cells

| Cell name | Tissue source | MFI (TINA) | MFI (hIgG1) | Positive rate (%) | Ratio |
|---|---|---|---|---|---|
| NCI-H322 | Non-small cell lung cancer cell | 2826998 | 21265 | 99.97 | 132.9 |
| Capan-1 | Human pancreatic cancer tumor cell | 1205346 | 65600 | 98.36 | 18.4 |
| PC-3 | Human prostate cancer cell | 68507 | 8925 | 69.09 | 7.7 |
| A549 | Human lung adenocarcinoma cell | 10105 | 9921 | 0.27 | 1.0 |
| HUVEC | Human umbilical vein endothelial cell | 125092 | 20475 | 22.86 | 6.1 |
| OVCAR-3 | Human ovarian cancer cell | 182074 | 11792 | 99.00 | 15.4 |
| HCC38 | Human ductal carcinoma cell | 3853305 | 32418 | 99.30 | 118.9 |
| HCC1806 | Human breast cancer cell | 1655211 | 16247 | 99.87 | 101.9 |

## Example 8. Antibody Binding Assay at Cellular Level

Tumor cells expressing Trop-2 were prepared into a cell suspension ($0.5$-$1 \times 10^6$ cells/mL) using the FACS buffer (Miltenyi Biotec, 130-091-221), and the cell suspension was added to a 96-well V-bottom plate at 100 μL/well. After centrifugation and removal of the supernatant, the test antibodies diluted with the FACS buffer to different concentrations (0.006 nM-100 nM) were each added at 50 μL/well. The mixture was incubated in the dark in a 4°C refrigerator for 1 h. The plate was then washed 3 times with the FACS buffer by centrifugation at 300 g, and Alexa Fluor 488 goat anti-human IgG (H+L) (Bio-legend, 409304, 1:500 dilution) at working concentration was added. The mixture was incubated in the dark in a 4°C refrigerator for 40 min. The plate was then washed 3 times with the FACS buffer by centrifugation at 300 g, the geometric mean fluorescence intensity (MFI) was measured on a Sartorius iQue3 flow cytometer, and the binding $EC_{50}$ values of the antibodies for tumor cells with different Trop-2 expression levels were calculated.

Table 7-1. Affinity of chimeric antibody and humanized antibodies of Ab2 for different tumor cells ($EC_{50}$)

| Antibody | HCC38 ($EC_{50}$, nM) | HCC1806 ($EC_{50}$, nM) |
|---|---|---|
| ch-Ab2 | 0.59 | 0.78 |
| hz-Ab2-1.2 | 0.76 | 0.90 |
| hz-Ab2-1.3 | 0.65 | 1.22 |
| hz-Ab2-1.4 | 1.20 | 1.07 |
| hz-Ab2-2.4 | 0.45 | 1.23 |
| TINA | 1.21 | 1.44 |

Table 7-2. Affinity of chimeric antibody and humanized antibodies of Ab21 for different tumor cells (EC$_{50}$)

| Antibody | HCC38 (EC$_{50}$,nM) | HCC1806 (EC$_{50}$,nM) | OVCAR-3 (EC$_{50}$,nM) |
|---|---|---|---|
| ch-Ab21 | 0.64 | 0.65 | 1.02 |
| hz-Ab21-1.1 | 0.55 | 0.64 | 0.62 |
| hz-Ab21-1.2 | 0.66 | 1.23 | 0.67 |
| hz-Ab21-2.1 | 0.64 | 0.63 | 0.68 |
| hz-Ab21-2.2 | 0.66 | 1.18 | 0.80 |
| TINA | 0.79 | 0.84 | 1.29 |

Table 7-3. Affinity of chimeric antibody and humanized antibodies of Ab35 for different tumor cells (EC$_{50}$)

| Antibody | HCC38 (EC$_{50}$,nM) | HCC1806 (EC$_{50}$,nM) | OVCAR-3 (EC$_{50}$,nM) |
|---|---|---|---|
| ch-Ab35 | 0.45 | 0.38 | 0.37 |
| hz-Ab35-1.1 | 0.77 | 0.80 | 0.83 |
| hz-Ab35-1.2 | 0.71 | 0.62 | 0.57 |
| hz-Ab35-1.3 | 0.70 | 0.54 | 0.64 |
| hz-Ab35-2.1 | 0.89 | 0.76 | 0.90 |
| hz-Ab35-2.2 | 0.91 | 0.58 | 0.88 |
| hz-Ab35-2.3 | 0.91 | 0.61 | 0.74 |
| TINA | 1.04 | 0.73 | 1.28 |

**Example 9. Data of Affinity Assays for Antibodies and Human Trop-2-His**

The affinity of antibodies for human Trop-2-His was detected in the form of antibody capturing. The antibody was affinity-captured by a CM5 (Cat. #29149603, GE) biosensor chip conjugated with an anti-human IgG antibody (from human antibody capture kit, Cat. #29234600, GE), then the antigen human Trop-2-His flowed on the surface of the chip, and a Biacore 8K instrument was used for detecting reaction signals in real time to obtain association and dissociation curves. After dissociation was completed for each assay cycle, the chip was washed clean and regenerated with a regeneration buffer, 3M MgCl$_2$ (from human antibody capture kit, Cat. #29234600, GE). The association and dissociation curves were collected by Biacore 8K Control Software 3.0 in real time, and the data were analyzed by Biacore Insight Evaluation Software 3.0. The data were fitted using a Langmuir 1: 1 model after double subtraction (namely, in each cycle, the contrast channel signal was subtracted from the experimental channel signal, and the sample signal was subtracted from the blank signal), and the association rate constant Ka, the dissociation rate constant Kd, and the equilibrium constant K$_D$ were calculated. The results show that all the humanized antibodies in the present application had a relatively high affinity (K$_D$ < 10$^{-8}$ M) for human Trop-2-His protein. See Table 8 for details.

Table 8. Affinity of humanized antibodies

| Antibody | Ka (1/Ms) | Kd (1/s) | K$_D$ (M) |
|---|---|---|---|
| hz-Ab2-1.1 | 6.06E+04 | 2.90E-03 | 4.78E-08 |
| hz-Ab2-1.2 | 3.56E+06 | 5.08E-03 | 1.43E-09 |
| hz-Ab2-1.3 | 1.00E+06 | 1.65E-02 | 1.65E-08 |
| hz-Ab2-1.4 | 1.22E+06 | 2.84E-03 | 2.32E-09 |
| hz-Ab2-2.1 | 3.36E+05 | 2.30E-04 | 6.86E-10 |

(continued)

| Antibody | Ka (1/Ms) | Kd (1/s) | $K_D$ (M) |
|---|---|---|---|
| hz-Ab2-2.2 | 2.45E+05 | 5.90E-04 | 2.41E-09 |
| hz-Ab2-2.3 | 2.37E+05 | 6.43E-04 | 2.72E-09 |
| hz-Ab2-2.4 | 4.15E+05 | 4.83E-03 | 1.16E-08 |
| hz-Ab21-1.1 | 4.29E+05 | 5.14E-04 | 1.20E-09 |
| hz-Ab21-1.2 | 4.50E+05 | 4.38E-04 | 9.73E-10 |
| hz-Ab21-2.1 | 4.04E+05 | 5.59E-04 | 1.38E-09 |
| hz-Ab21-2.2 | 4.60E+05 | 6.11E-04 | 1.33E-09 |
| hz-Ab35-1.1 | 3.79E+05 | 2.38E-04 | 6.28E-10 |
| hz-Ab35-1.2 | 4.01E+05 | 3.88E-03 | 9.66E-09 |
| hz-Ab35-1.3 | 4.17E+05 | 3.10E-04 | 7.43E-10 |
| hz-Ab35-2.1 | 4.36E+05 | 1.94E-04 | 4.44E-10 |
| hz-Ab35-2.2 | 4.05E+05 | 3.57E-03 | 8.82E-09 |
| hz-Ab35-2.3 | 4.16E+05 | 2.81E-04 | 6.75E-10 |
| TINA | 1.91E+05 | 2.39E-03 | 1.25E-08 |

## Example 10. Detection of Cross-Activity Between Antibodies and Monkey-Derived Protein

Macaque Trop-2-His (specifically, the extracellular domain of the macaque Trop-2 was recombinantly linked to His, SEQ ID NO: 4) was diluted to 2 $\mu$g/mL with PBS (SmartBuffer, PBS-2050) buffer at pH 7.4, added into a 96-well microplate at 100 $\mu$L/well, and incubated overnight at 4°C. After the liquid was discarded, 2% BSA (SIGMA, B2064-100G) diluted with PBS was added at 300 $\mu$L/well for blocking, and the mixture was incubated at 37°C for 2 h. After the blocking was completed, the blocking buffer was discarded, the plate was washed 3 times with the PBST buffer (pH 7.4 PBS containing 0.1% tween-20), the antibody solution diluted in a gradient was added at 100 $\mu$L/well, and the mixture was incubated at 37°C for 1 h. After the incubation was completed, the plate was washed 3 times with PBST, the goat anti-human IgG (H+L) (JacksonImmunoResearch, 109-035-098, 1:5000 dilution) was added at 100 $\mu$L/well, and the mixture was incubated at 37°C for 1 h. After the plate was washed 3 times with PBST, a TMB chromogenic substrate (TIANGEN, PA107-01) was added at 100 $\mu$L/well. The mixture was incubated at room temperature for 10-15 min, and then 1 M $H_2SO_4$ was added at 50 $\mu$L/well to terminate the reaction. The absorbance values at 450 nm were taken using a microplate reader. The binding curves of antibodies for antigen were fitted using software, and the $EC_{50}$ values were calculated. The assay data indicate that all the humanized antibodies of the present application had relatively good binding activity for monkey Trop-2.

Table 9-1. Binding activity of chimeric antibody and humanized antibodies of Ab2 for monkey Trop-2-His protein

| Humanized antibody | Affinity, $EC_{50}$ (nM) |
|---|---|
| ch-Ab2 | 0.048 |
| hz-Ab2-1.1 | 0.133 |
| hz-Ab2-1.2 | 0.064 |
| hz-Ab2-1.3 | 0.068 |
| hz-Ab2-1.4 | 0.053 |
| hz-Ab2-2.1 | 1.188 |
| hz-Ab2-2.2 | 0.341 |
| hz-Ab2-2.3 | 0.830 |
| hz-Ab2-2.4 | 0.070 |

(continued)

| Humanized antibody | Affinity, $EC_{50}$ (nM) |
|---|---|
| TINA | 0.106 |

Table 9-2. Binding activity of chimeric antibody and humanized antibodies of Ab21 for monkey Trop-2-His protein

| Humanized antibody | Affinity, $EC_{50}$ (nM) |
|---|---|
| ch-Ab21 | 0.093 |
| hz-Ab21-1.1 | 0.098 |
| hz-Ab21-1.2 | 0.086 |
| hz-Ab21-2.1 | 0.064 |
| hz-Ab21-2.2 | 0.081 |
| TINA | 0.063 |

Table 9-3. Binding activity of chimeric antibody and humanized antibodies of Ab35 for monkey Trop-2-His protein

| Humanized antibody | Affinity, $EC_{50}$ (nM) |
|---|---|
| ch-Ab35 | 0.048 |
| hz-Ab35-1.1 | 0.047 |
| hz-Ab35-1.2 | 0.038 |
| hz-Ab35-1.3 | 0.033 |
| hz-Ab35-2.1 | 0.056 |
| hz-Ab35-2.2 | 0.050 |
| hz-Ab35-2.3 | 0.031 |
| TINA | 0.046 |

**Example 11. Non-Specific Binding of Antibodies in Tumor Cells Not Expressing Trop-2**

Tumor cells A549 (lung cancer tumor cells) not expressing Trop-2 were prepared into a cell suspension (0.5-1 $\times 10^6$ cells/mL) using the FACS buffer (Miltenyi Biotec, 130-091-221), and the cell suspension was added to a 96-well V-bottom plate at 100 $\mu$L/well. After centrifugation and removal of the supernatant, the test antibodies diluted with the FACS buffer to different concentrations (0.0006 nM-50 nM) were each added at 50 $\mu$L/well. The mixture was incubated in the dark in a 4°C refrigerator for 1 h. The plate was then washed 3 times with the FACS buffer by centrifugation at 300 g, and Alexa Fluor 488 goat anti-human IgG (H+L) (Bio-legend, 409304, 1:500 dilution) at working concentration was added. The mixture was incubated in the dark in a 4°C refrigerator for 40 min. The plate was then washed 3 times with the FACS buffer by centrifugation at 300 g, and the geometric mean fluorescence intensity (MFI) was measured on a Sartorius iQue3 flow cytometer. The results in FIG. 2 show that the humanized antibodies of the present application did not non-specifically bind to lung cancer tumor cells A549 not expressing Trop-2.

**Example 12. Experiment on Antibody Internalization in Tumor Cells**

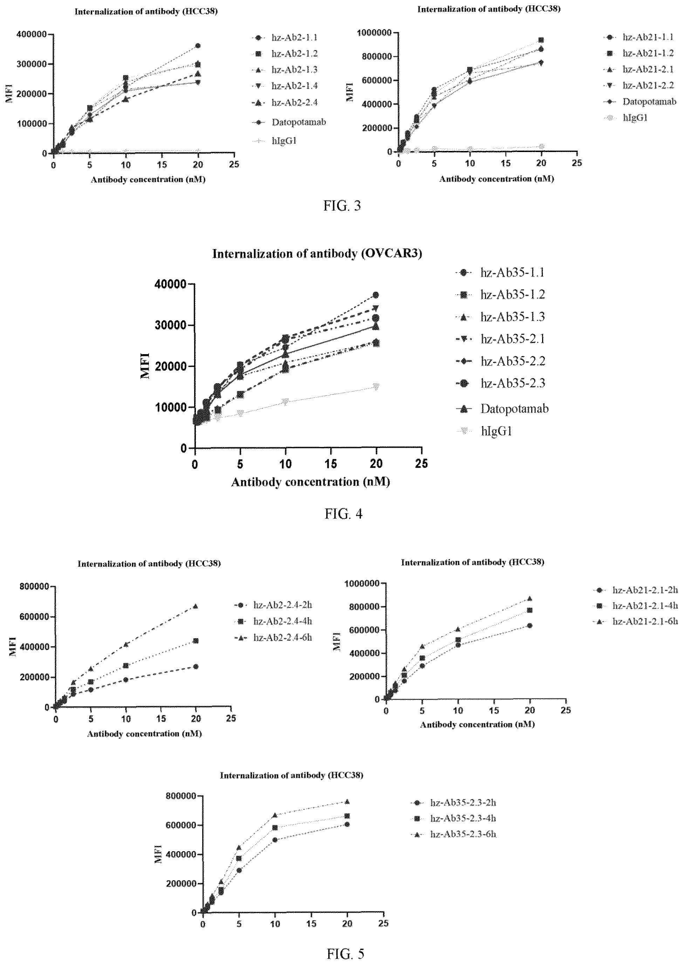

The internalization of antibodies in the tumor cells was detected by using an antibody internalization kit (Sartoius, 90564). Firstly, the labeled antibody in the kit and the test humanized or chimeric antibody were each diluted to 40 nM by using a complete medium used for detecting the cells, then the labeled antibody and the humanized or chimeric antibody, at their initial concentrations, were mixed in a 96-well V-bottom cell culture plate (Corning, Cat. No.: 3894) at a ratio of 1:1, and then incubated at 37°C for different time periods (2 h, 4 h, or 6 h) after 2-fold dilution. The geometric mean fluorescence intensity (MFI) was detected on a Sartorius iQue3 flow cytometer, wherein the stronger the geometric mean fluorescence intensity (MFI), the stronger the internalization of the antibody in the cell. The results show that the humanized antibodies of the present application exhibited relatively great internalization activity in cell lines with varying Trop-2 expression amounts, and the amount of internalized antibody accumulated with increasing internalization time without exhibiting significant saturation at a relatively high concentration of antibody (20 nM). The assay results are shown in FIGs. 3-6.

**Example 13. ADCC Activity of Anti-Trop-2 Antibodies**

Based on the reporter gene method, the antibody-dependent cell-mediated cytotoxicity (ADCC) activity of anti-Trop-2 antibodies was determined using NCI-H322 and HCC1806 as target cells and Jurkat cell stably transfected with CD16a v158 and NFAT-luciferase (Jurkat-CD16a v158-NFAT-luciferase) (BPS Bioscience, Cat. No.: 60541) as the effector cell. TINA was used as the positive control, and a hIgG1 isotype control antibody (Biointron, Cat. No.: B117901) was used as the negative control.

NCI-H322 and HCC1806 cells were each diluted to $3 \times 10^5$ cells/mL with medium, and each cell suspension was added to a 96-well plate at 30 $\mu$L/well, and then test antibodies at different concentrations were each added to the 96-well plate at 40 $\mu$L/well, so that the final concentrations of the test antibodies were 40 nM, 8 nM, 1.6 nM, 0.32 nM, 0.064 nM, 0.013 nM, 0.0026 nM, and 0.0005 nM. Jurkat-CD16a v158-NFAT-luciferase cells were then taken out; the Jurkat-CD16a v158-NFAT-luciferase cell suspension was added to the 96-well plate at 30 $\mu$L/well based on an effector-to-target ratio of 10:1. Centrifugation at low speed was performed to allow the effector cells and the target cells to be sufficiently in contact with each other and mixed, and the mixture was incubated in an incubator at 37°C with 5% $CO_2$ for 5-6 h. The detection reagent was then added at 100 $\mu$L/well according to the instructions of the firefly luciferase detection kit (Vazyme, Cat. No.: DD1203-03), and the mixture was left to stand for 15-30 min, and the fluorescence signals were detected by using a microplate reader (Thermo, Varioskan Flash).

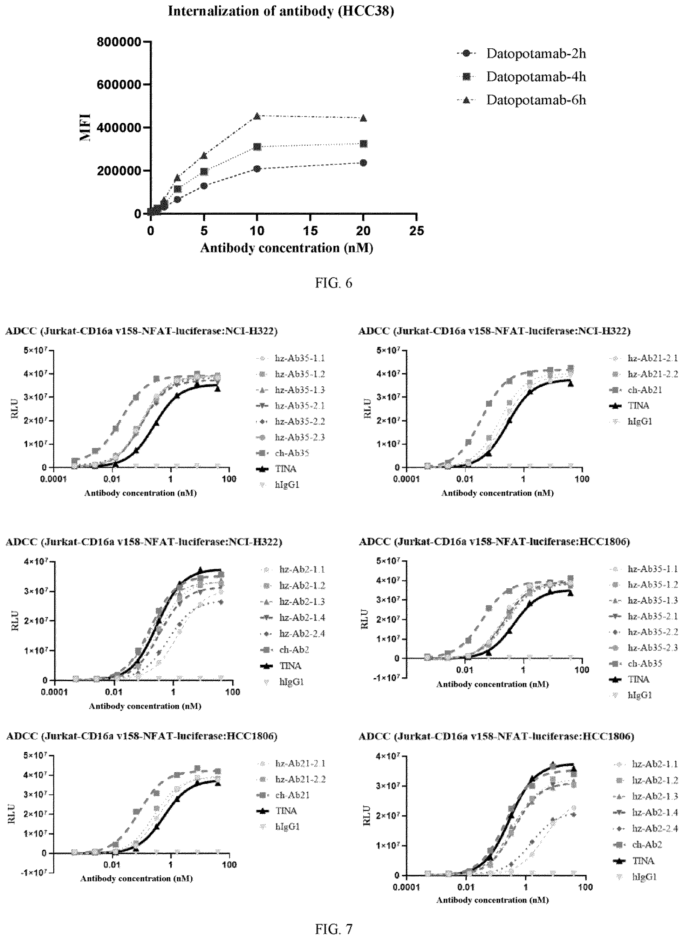

In reporter gene experiments, the ADCC activity of the anti-Trop-2 antibodies is shown in FIG. 7, and the $EC_{50}$ data are shown in Table 10. The chimeric antibodies ch-Ab2, ch-Ab21 and ch-Ab35 and humanized antibodies hz-Ab2-1.2, hz-Ab2-1.4, hz-Ab2-1.3, hz-Ab21-2.1, hz-Ab21-2.2, hz-Ab35-1.1, hz-Ab35-1.2, hz-Ab35-1.3, hz-Ab35-2.1, hz-Ab35-2.2, and hz-Ab35-2.3 were all able to significantly induce the activation of the NFAT signal of Jurkat-CD16a v 15 8- NF AT-luciferase cells on two different cells and were better than TINA.

Table 10. ADCC Activity of anti-Trop-2 antibodies (with Jurkat-CD16a v158-NFAT-luciferase as effector cell

| Antibody | $EC_{50}$ (nM) | |
|---|---|---|
| | H322 | HCC1806 |
| ch-Ab2 | 0.17 | 0.20 |
| ch-Ab21 | 0.04 | 0.08 |
| ch-Ab35 | 0.02 | 0.04 |
| hz-Ab2-1.1 | 1.45 | 4.10 |
| hz-Ab2-1.2 | 0.31 | 0.42 |
| hz-Ab2-1.3 | 0.17 | 0.33 |
| hz-Ab2-1.4 | 0.34 | 0.39 |
| hz-Ab2-2.4 | 0.66 | 1.51 |
| hz-Ab21-2.1 | 0.21 | 0.39 |
| hz-Ab21-2.2 | 0.15 | 0.26 |
| hz-Ab35-1.1 | 0.09 | 0.18 |
| hz-Ab35-1.2 | 0.10 | 0.28 |
| hz-Ab35-1.3 | 0.11 | 0.20 |
| hz-Ab35-2.1 | 0.10 | 0.23 |
| hz-Ab35-2.2 | 0.11 | 0.26 |
| hz-Ab35-2.3 | 0.11 | 0.21 |

(continued)

| Antibody | EC$_{50}$ (nM) | |
|---|---|---|
| | H322 | HCC1806 |
| TINA | 0.27 | 0.46 |

Based on the killing effect of PBMC, the ADCC activity of the anti-Trop-2 antibodies was detected using NCI-H322 and HCC1806 as the target cells and PBMC as the effector cell. TINA was used as the positive control, and a hIgG 1 isotype control antibody (Biointron, Cat. No.: B117901) was used as the negative control.

NCI-H322 and HCC1806 cells were each diluted to $3 \times 10^5$ cells/mL with the medium, and each cell suspension was added to a 96-well plate at 50 $\mu$L/well. Then, test antibodies at different concentrations were added to the wells of the 96-well plate, so that the final concentrations of the test antibodies were 66.7 nM, 13.34 nM, 2.67 nM, 0.53 nM, 0.11 nM, 0.021 nM, 0.004 nM, and 0.0008 nM. PBMC cells were thawed; the PBMC cell suspension (cell density of $1.5 \times 10^6$ cells/mL) was added to the 96-well plate at 100 $\mu$L/well based on an effector-to-target ratio of 10:1. Centrifugation at low speed was performed to allow the effector cells and the target cells to be sufficiently in contact with each other and mixed, and the mixture was incubated in an incubator at 37°C with 5% $CO_2$ for 18 h. For the lysis buffer group and the maximum release group, a lysis buffer was added at 20 $\mu$L/well. The mixture was incubated in an incubator at 37°C with 5% $CO_2$ for 45 min, then the 96-well plate was placed in a plate centrifuge and centrifuged at 250 g/min for 5 min, and 50 $\mu$L of supernatant was taken from each well and added to a 96-well detection plate. The LDH working solution was prepared according to the instructions of the CytoTox96 Non-Radio kit (Promega, Cat. No.: PR-G1781) and then added to the 96-well detection plate at 50 $\mu$L/well, and after sufficient mixing, the mixture was incubated in the dark at room temperature for 25-35 min. The stop solution (Promega, Cat. No.: PR-G1782) was added at 50 $\mu$L/well, and after sufficient mixing, OD values at a wavelength of 492 nm were read using a microplate reader (Tecan, Infinite F50).

$$\text{Killing rate (\%)} = \textbf{Fehler!} \times 100\%$$

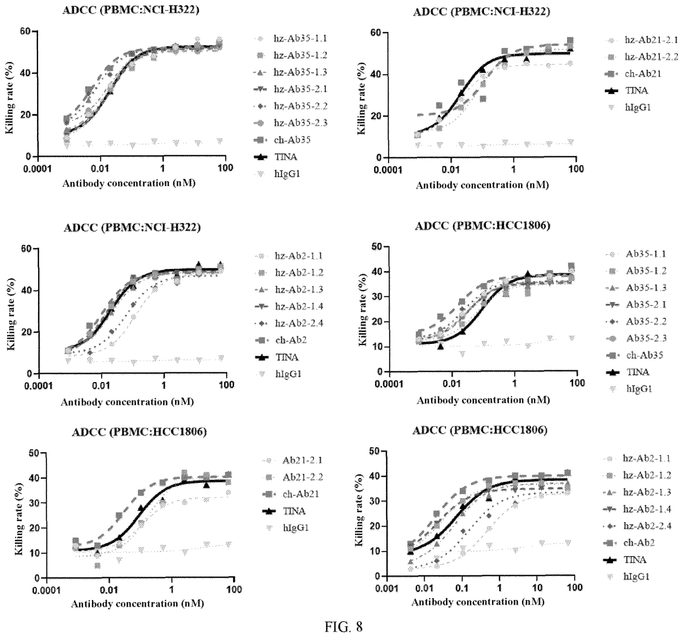

As shown in FIG. 8 and Table 11, in PBMC killing experiments, the ADCC activity of anti-Trop-2 antibodies ch-Ab2, ch-Ab21 and ch-Ab35 and humanized antibodies hz-Ab2-1.2, hz-Ab2-1.3, hz-Ab2-1.4, hz-Ab35-1.1, hz-Ab35-1.2, hz-Ab35-1.3, hz-Ab35-2.1, and hz-Ab35-2.2 was better than or comparable to TINA at two different cell levels.

Table 11. ADCC activity of anti-**Trop-2** chimeric antibodies (with PBMC as the effector cell)

| Antibody | H322 | | HCC1806 | |
|---|---|---|---|---|
| | EC$_{50}$ (nM) | Maximum killing (%) | EC$_{50}$ (nM) | Maximum killing (%) |
| ch-Ab2 | 0.01 | 48.14 | 0.02 | 40.02 |
| ch-Ab21 | 0.13 | 53.96 | 0.03 | 40.29 |
| ch-Ab35 | 0.01 | 51.93 | 0.02 | 38.03 |
| hz-Ab2-1.1 | 0.13 | 47.82 | 0.46 | 32.92 |
| hz-Ab2-1.2 | 0.01 | 48.37 | 0.03 | 36.62 |
| hz-Ab2-1.3 | 0.02 | 48.13 | 0.06 | 36.97 |
| hz-Ab2-1.4 | 0.01 | 48.67 | 0.02 | 34.82 |
| hz-Ab2-2.4 | 0.07 | 46.70 | 0.15 | 33.34 |
| hz-Ab21-2.1 | 0.02 | 44.54 | 0.12 | 32.24 |
| hz-Ab21-2.2 | 0.07 | 51.63 | 0.18 | 40.58 |
| hz-Ab35-1.1 | 0.02 | 53.00 | 0.04 | 38.53 |
| hz-Ab35-1.2 | 0.02 | 51.46 | 0.06 | 34.87 |
| hz-Ab35-1.3 | 0.02 | 51.55 | 0.04 | 35.72 |
| hz-Ab35-2.1 | 0.02 | 51.28 | 0.03 | 35.18 |
| hz-Ab35-2.2 | 0.01 | 51.37 | 0.02 | 35.87 |

(continued)

| Antibody | H322 | | HCC1806 | |
|---|---|---|---|---|
| | EC$_{50}$ (nM) | Maximum killing (%) | EC$_{50}$ (nM) | Maximum killing (%) |
| hz-Ab35-2.3 | 0.02 | 50.29 | 0.03 | 35.68 |
| TINA | 0.02 | 49.75 | 0.07 | 38.45 |

According to the content disclosed in the present application, the methods of the present application have been described in terms of preferred embodiments, but it will be apparent to those skilled in the art that variations may be applied to the methods and the steps or the sequence of steps of the methods described herein without departing from the concept, spirit, and scope of the present application, and all these shall fall within the protection scope of the present disclosure.

The disclosed content of all documents cited herein are hereby incorporated by reference to the extent that they provide exemplary, procedural, and additional details supplementary to those described herein. These publications are provided solely because they were disclosed prior to the filing date of the present application. All statements as to the dates of these documents or descriptions as to the content of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

**Claims**

1. An antibody or an antigen-binding fragment thereof binding to human TROP-2, comprising a heavy chain variable region and a light chain variable region, wherein the heavy chain variable region comprises a heavy chain CDR1, a heavy chain CDR2, and a heavy chain CDR3 in the variable region sequence set forth in SEQ ID NO: 65, SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63 or SEQ ID NO: 64, and the light chain variable region comprises a light chain CDR1, a light chain CDR2, and a light chain CDR3 in the variable region sequence set forth in SEQ ID NO: 66, SEQ ID NO: 24, SEQ ID NO: 26, SEQ ID NO: 28, SEQ ID NO: 40, SEQ ID NO: 41, SEQ ID NO: 54, SEQ ID NO: 55, or SEQ ID NO: 67.

2. The antibody or the antigen-binding fragment thereof binding to human TROP-2 according to claim 1, wherein

   the heavy chain variable region comprises the heavy chain CDR1, the heavy chain CDR2, and the heavy chain CDR3 in the variable region sequence set forth in SEQ ID NO: 65, and the light chain variable region comprises the light chain CDR1, the light chain CDR2, and the light chain CDR3 in the variable region sequence set forth in SEQ ID NO: 66;
   the heavy chain variable region comprises the heavy chain CDR1, the heavy chain CDR2, and the heavy chain CDR3 in the variable region sequence set forth in SEQ ID NO: 23, and the light chain variable region comprises the light chain CDR1, the light chain CDR2, and the light chain CDR3 in the variable region sequence set forth in SEQ ID NO: 24;
   the heavy chain variable region comprises the heavy chain CDR1, the heavy chain CDR2, and the heavy chain CDR3 in the variable region sequence set forth in SEQ ID NO: 25, and the light chain variable region comprises the light chain CDR1, the light chain CDR2, and the light chain CDR3 in the variable region sequence set forth in SEQ ID NO: 26;
   the heavy chain variable region comprises the heavy chain CDR1, the heavy chain CDR2, and the heavy chain CDR3 in the variable region sequence set forth in SEQ ID NO: 27, and the light chain variable region comprises the light chain CDR1, the light chain CDR2, and the light chain CDR3 in the variable region sequence set forth in SEQ ID NO: 28;
   the heavy chain variable region comprises the heavy chain CDR1, the heavy chain CDR2, and the heavy chain CDR3 in the variable region sequence set forth in SEQ ID NO: 36, and the light chain variable region comprises the light chain CDR1, the light chain CDR2, and the light chain CDR3 in the variable region sequence set forth in SEQ ID NO: 40;
   the heavy chain variable region comprises the heavy chain CDR1, the heavy chain CDR2, and the heavy chain CDR3 in the variable region sequence set forth in SEQ ID NO: 37, and the light chain variable region comprises the light chain CDR1, the light chain CDR2, and the light chain CDR3 in the variable region sequence set forth in SEQ ID NO: 40;

the heavy chain variable region comprises the heavy chain CDR1, the heavy chain CDR2, and the heavy chain CDR3 in the variable region sequence set forth in SEQ ID NO: 38, and the light chain variable region comprises the light chain CDR1, the light chain CDR2, and the light chain CDR3 in the variable region sequence set forth in SEQ ID NO: 40;

the heavy chain variable region comprises the heavy chain CDR1, the heavy chain CDR2, and the heavy chain CDR3 in the variable region sequence set forth in SEQ ID NO: 39, and the light chain variable region comprises the light chain CDR1, the light chain CDR2, and the light chain CDR3 in the variable region sequence set forth in SEQ ID NO: 40;

the heavy chain variable region comprises the heavy chain CDR1, the heavy chain CDR2, and the heavy chain CDR3 in the variable region sequence set forth in SEQ ID NO: 36, and the light chain variable region comprises the light chain CDR1, the light chain CDR2, and the light chain CDR3 in the variable region sequence set forth in SEQ ID NO: 41;

the heavy chain variable region comprises the heavy chain CDR1, the heavy chain CDR2, and the heavy chain CDR3 in the variable region sequence set forth in SEQ ID NO: 37, and the light chain variable region comprises the light chain CDR1, the light chain CDR2, and the light chain CDR3 in the variable region sequence set forth in SEQ ID NO: 41;

the heavy chain variable region comprises the heavy chain CDR1, the heavy chain CDR2, and the heavy chain CDR3 in the variable region sequence set forth in SEQ ID NO: 38, and the light chain variable region comprises the light chain CDR1, the light chain CDR2, and the light chain CDR3 in the variable region sequence set forth in SEQ ID NO: 41;

the heavy chain variable region comprises the heavy chain CDR1, the heavy chain CDR2, and the heavy chain CDR3 in the variable region sequence set forth in SEQ ID NO: 39, and the light chain variable region comprises the light chain CDR1, the light chain CDR2, and the light chain CDR3 in the variable region sequence set forth in SEQ ID NO: 41;

the heavy chain variable region comprises the heavy chain CDR1, the heavy chain CDR2, and the heavy chain CDR3 in the variable region sequence set forth in SEQ ID NO: 50, and the light chain variable region comprises the light chain CDR1, the light chain CDR2, and the light chain CDR3 in the variable region sequence set forth in SEQ ID NO: 54;

the heavy chain variable region comprises the heavy chain CDR1, the heavy chain CDR2, and the heavy chain CDR3 in the variable region sequence set forth in SEQ ID NO: 51, and the light chain variable region comprises the light chain CDR1, the light chain CDR2, and the light chain CDR3 in the variable region sequence set forth in SEQ ID NO: 54;

the heavy chain variable region comprises the heavy chain CDR1, the heavy chain CDR2, and the heavy chain CDR3 in the variable region sequence set forth in SEQ ID NO: 52, and the light chain variable region comprises the light chain CDR1, the light chain CDR2, and the light chain CDR3 in the variable region sequence set forth in SEQ ID NO: 54;

the heavy chain variable region comprises the heavy chain CDR1, the heavy chain CDR2, and the heavy chain CDR3 in the variable region sequence set forth in SEQ ID NO: 53, and the light chain variable region comprises the light chain CDR1, the light chain CDR2, and the light chain CDR3 in the variable region sequence set forth in SEQ ID NO: 54;

the heavy chain variable region comprises the heavy chain CDR1, the heavy chain CDR2, and the heavy chain CDR3 in the variable region sequence set forth in SEQ ID NO: 50, and the light chain variable region comprises the light chain CDR1, the light chain CDR2, and the light chain CDR3 in the variable region sequence set forth in SEQ ID NO: 55;

the heavy chain variable region comprises the heavy chain CDR1, the heavy chain CDR2, and the heavy chain CDR3 in the variable region sequence set forth in SEQ ID NO: 51, and the light chain variable region comprises the light chain CDR1, the light chain CDR2, and the light chain CDR3 in the variable region sequence set forth in SEQ ID NO: 55;

the heavy chain variable region comprises the heavy chain CDR1, the heavy chain CDR2, and the heavy chain CDR3 in the variable region sequence set forth in SEQ ID NO: 52, and the light chain variable region comprises the light chain CDR1, the light chain CDR2, and the light chain CDR3 in the variable region sequence set forth in SEQ ID NO: 55;

the heavy chain variable region comprises the heavy chain CDR1, the heavy chain CDR2, and the heavy chain CDR3 in the variable region sequence set forth in SEQ ID NO: 53, and the light chain variable region comprises the light chain CDR1, the light chain CDR2, and the light chain CDR3 in the variable region sequence set forth in SEQ ID NO: 55;

the heavy chain variable region comprises the heavy chain CDR1, the heavy chain CDR2, and the heavy chain CDR3 in the variable region sequence set forth in SEQ ID NO: 63, and the light chain variable region comprises the light chain CDR1, the light chain CDR2, and the light chain CDR3 in the variable region sequence set forth in SEQ ID NO: 66;

the heavy chain variable region comprises the heavy chain CDR1, the heavy chain CDR2, and the heavy chain CDR3 in the variable region sequence set forth in SEQ ID NO: 64, and the light chain variable region comprises the light chain CDR1, the light chain CDR2, and the light chain CDR3 in the variable region sequence set forth in SEQ ID NO: 66;

the heavy chain variable region comprises the heavy chain CDR1, the heavy chain CDR2, and the heavy chain CDR3 in the variable region sequence set forth in SEQ ID NO: 63, and the light chain variable region comprises the light chain CDR1, the light chain CDR2, and the light chain CDR3 in the variable region sequence set forth in SEQ ID NO: 67;

the heavy chain variable region comprises the heavy chain CDR1, the heavy chain CDR2, and the heavy chain CDR3 in the variable region sequence set forth in SEQ ID NO: 64, and the light chain variable region comprises the light chain CDR1, the light chain CDR2, and the light chain CDR3 in the variable region sequence set forth in SEQ ID NO: 67; or

the heavy chain variable region comprises the heavy chain CDR1, the heavy chain CDR2, and the heavy chain CDR3 in the variable region sequence set forth in SEQ ID NO: 65, and the light chain variable region comprises the light chain CDR1, the light chain CDR2, and the light chain CDR3 in the variable region sequence set forth in SEQ ID NO: 67.

3. An antibody or an antigen-binding fragment thereof binding to human TROP-2, comprising a heavy chain variable region and a light chain variable region, wherein the heavy chain variable region comprises a heavy chain CDR1, a heavy chain CDR2, and a heavy chain CDR3, the heavy chain CDR1 having at least 80% identity to an amino acid sequence selected from the group consisting of SEQ ID NO: 17, SEQ ID NO: 5, SEQ ID NO: 11, and SEQ ID NO: 48, the heavy chain CDR2 having at least 80% identity to an amino acid sequence selected from the group consisting of SEQ ID NO: 18, SEQ ID NO: 6, SEQ ID NO: 31, SEQ ID NO: 32, SEQ ID NO: 49, SEQ ID NO: 12, and SEQ ID NO: 62, and the heavy chain CDR3 having at least 80% identity to an amino acid sequence selected from the group consisting of SEQ ID NO: 19, SEQ ID NO: 7, and SEQ ID NO: 13; the light chain variable region comprises a light chain CDR1, a light chain CDR2, and a light chain CDR3, the light chain CDR1 having at least 80% identity to an amino acid sequence selected from the group consisting of SEQ ID NO: 20, SEQ ID NO: 8, SEQ ID NO: 33, and SEQ ID NO: 14, the light chain CDR2 having at least 80% identity to an amino acid sequence selected from the group consisting of SEQ ID NO: 21, SEQ ID NO: 9, SEQ ID NO: 34, and SEQ ID NO: 15, and the light chain CDR3 having at least 80% identity to an amino acid sequence selected from the group consisting of SEQ ID NO: 22, SEQ ID NO: 10, SEQ ID NO: 35, and SEQ ID NO: 16.

4. The antibody or the antigen-binding fragment thereof according to claim 3, wherein the heavy chain CDR1, the heavy chain CDR2, the heavy chain CDR3, the light chain CDR1, the light chain CDR2, and the light chain CDR3 are selected from the group consisting of the following sequences or sequences having at least 80% identity thereto, respectively:

|  |  |  |
|---|---|---|
| i. | heavy chain CDR1 | SEQ ID NO: 17, |
|  | heavy chain CDR2 | SEQ ID NO: 18, |
|  | heavy chain CDR3 | SEQ ID NO: 19, |
|  | light chain CDR1 | SEQ ID NO: 20, |
|  | light chain CDR2 | SEQ ID NO: 21, |
|  | light chain CDR3 | SEQ ID NO: 22; or, |
| 11. | heavy chain CDR1 | SEQ ID NO: 5, |
|  | heavy chain CDR2 | SEQ ID NO: 32, |
|  | heavy chain CDR3 | SEQ ID NO: 7, |
|  | light chain CDR1 | SEQ ID NO: 8, |
|  | light chain CDR2 | SEQ ID NO: 34, |
|  | light chain CDR3 | SEQ ID NO: 10; or, |
| iii. | heavy chain CDR1 | SEQ ID NO: 5, |
|  | heavy chain CDR2 | SEQ ID NO: 31, |

(continued)

|       |                  |                    |
|-------|------------------|--------------------|
|       | heavy chain CDR3 | SEQ ID NO: 7,      |
|       | light chain CDR1 | SEQ ID NO: 33,     |
|       | light chain CDR2 | SEQ ID NO: 34,     |
|       | light chain CDR3 | SEQ ID NO: 35; or, |
| iv.   | heavy chain CDR1 | SEQ ID NO: 5,      |
|       | heavy chain CDR2 | SEQ ID NO: 32,     |
|       | heavy chain CDR3 | SEQ ID NO: 7,      |
|       | light chain CDR1 | SEQ ID NO: 33,     |
|       | light chain CDR2 | SEQ ID NO: 34,     |
|       | light chain CDR3 | SEQ ID NO: 35; or, |
| v.    | heavy chain CDR1 | SEQ ID NO: 48,     |
|       | heavy chain CDR2 | SEQ ID NO: 49,     |
|       | heavy chain CDR3 | SEQ ID NO: 13,     |
|       | light chain CDR1 | SEQ ID NO: 14,     |
|       | light chain CDR2 | SEQ ID NO: 15,     |
|       | light chain CDR3 | SEQ ID NO: 16; or, |
| vi.   | heavy chain CDR1 | SEQ ID NO: 48,     |
|       | heavy chain CDR2 | SEQ ID NO: 12,     |
|       | heavy chain CDR3 | SEQ ID NO: 13,     |
|       | light chain CDR1 | SEQ ID NO: 14,     |
|       | light chain CDR2 | SEQ ID NO: 15,     |
|       | light chain CDR3 | SEQ ID NO: 16; or, |
| vii.  | heavy chain CDR1 | SEQ ID NO: 17,     |
|       | heavy chain CDR2 | SEQ ID NO: 62,     |
|       | heavy chain CDR3 | SEQ ID NO: 19,     |
|       | light chain CDR1 | SEQ ID NO: 20,     |
|       | light chain CDR2 | SEQ ID NO: 21,     |
|       | light chain CDR3 | SEQ ID NO: 22; or, |
| viii. | heavy chain CDR1 | SEQ ID NO: 17,     |
|       | heavy chain CDR2 | SEQ ID NO: 18,     |
|       | heavy chain CDR3 | SEQ ID NO: 19,     |
|       | light chain CDR1 | SEQ ID NO: 20,     |
|       | light chain CDR2 | SEQ ID NO: 21,     |
|       | light chain CDR3 | SEQ ID NO: 22; or, |
| ix.   | heavy chain CDR1 | SEQ ID NO: 5,      |
|       | heavy chain CDR2 | SEQ ID NO: 6,      |
|       | heavy chain CDR3 | SEQ ID NO: 7,      |
|       | light chain CDR1 | SEQ ID NO: 8,      |
|       | light chain CDR2 | SEQ ID NO: 9,      |
|       | light chain CDR3 | SEQ ID NO: 10; or, |
| x.    | heavy chain CDR1 | SEQ ID NO: 11,     |
|       | heavy chain CDR2 | SEQ ID NO: 12,     |
|       | heavy chain CDR3 | SEQ ID NO: 13,     |
|       | light chain CDR1 | SEQ ID NO: 14,     |
|       | light chain CDR2 | SEQ ID NO: 15,     |
|       | light chain CDR3 | SEQ ID NO: 16; or, |
| xi.   | heavy chain CDR1 | SEQ ID NO: 5,      |
|       | heavy chain CDR2 | SEQ ID NO: 31,     |
|       | heavy chain CDR3 | SEQ ID NO: 7,      |
|       | light chain CDR1 | SEQ ID NO: 8,      |
|       | light chain CDR2 | SEQ ID NO: 34,     |

(continued)

light chain CDR3     SEQ ID NO: 10.

5. The antibody or the antigen-binding fragment thereof according to any one of claims 1-4, wherein the heavy chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 65, SEQ ID NO: 23, SEQ ID NO: 25, SEQ ID NO: 27, SEQ ID NO: 36, SEQ ID NO: 37, SEQ ID NO: 38, SEQ ID NO: 39, SEQ ID NO: 50, SEQ ID NO: 51, SEQ ID NO: 52, SEQ ID NO: 53, SEQ ID NO: 63, and SEQ ID NO: 64, and wherein the light chain variable region has at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, or at least 99% identity to an amino acid sequence selected from the group consisting of SEQ ID NO: 66, SEQ ID NO: 24, SEQ ID NO: 26, SEQ ID NO: 28, SEQ ID NO: 40, SEQ ID NO: 41, SEQ ID NO: 54, SEQ ID NO: 55, and SEQ ID NO: 67.

6. The antibody or the antigen-binding fragment thereof according to claim 5, wherein the heavy chain variable region and the light chain variable region are selected from the group consisting of the following sequences or sequences having at least 80% identity thereto, respectively:

i) heavy chain variable region SEQ ID NO: 65, light chain variable region SEQ ID NO: 66; or
ii) heavy chain variable region SEQ ID NO: 37, light chain variable region SEQ ID NO: 40; or
iii) heavy chain variable region SEQ ID NO: 38, light chain variable region SEQ ID NO: 40; or
iv) heavy chain variable region SEQ ID NO: 39, light chain variable region SEQ ID NO: 40; or
v) heavy chain variable region SEQ ID NO: 36, light chain variable region SEQ ID NO: 41; or
vi) heavy chain variable region SEQ ID NO: 37, light chain variable region SEQ ID NO: 41; or
vii) heavy chain variable region SEQ ID NO: 38, light chain variable region SEQ ID NO: 41; or
viii) heavy chain variable region SEQ ID NO: 39, light chain variable region SEQ ID NO: 41; or
ix) heavy chain variable region SEQ ID NO: 50, light chain variable region SEQ ID NO: 54; or
x) heavy chain variable region SEQ ID NO: 51, light chain variable region SEQ ID NO: 54; or
xi) heavy chain variable region SEQ ID NO: 52, light chain variable region SEQ ID NO: 54; or
xii) heavy chain variable region SEQ ID NO: 53, light chain variable region SEQ ID NO: 54; or
xiii) heavy chain variable region SEQ ID NO: 50, light chain variable region SEQ ID NO: 55; or
xiv) heavy chain variable region SEQ ID NO: 51, light chain variable region SEQ ID NO: 55; or
xv) heavy chain variable region SEQ ID NO: 52, light chain variable region SEQ ID NO: 55; or
xvi) heavy chain variable region SEQ ID NO: 53, light chain variable region SEQ ID NO: 55; or
xvii) heavy chain variable region SEQ ID NO: 63, light chain variable region SEQ ID NO: 66; or
xviii) heavy chain variable region SEQ ID NO: 64, light chain variable region SEQ ID NO: 66; or
xix) heavy chain variable region SEQ ID NO: 36, light chain variable region SEQ ID NO: 40; or
xx) heavy chain variable region SEQ ID NO: 63, light chain variable region SEQ ID NO: 67; or
xxi) heavy chain variable region SEQ ID NO: 64, light chain variable region SEQ ID NO: 67; or
xxii) heavy chain variable region SEQ ID NO: 65, light chain variable region SEQ ID NO: 67; or
xxiii) heavy chain variable region SEQ ID NO: 23, light chain variable region SEQ ID NO: 24; or
xxiv) heavy chain variable region SEQ ID NO: 25, light chain variable region SEQ ID NO: 26; or
xxv) heavy chain variable region SEQ ID NO: 27, light chain variable region SEQ ID NO: 28.

7. The antibody or the antigen-binding fragment thereof according to claim 6, comprising a heavy chain and a light chain, wherein the heavy chain and the light chain are selected from the group consisting of the following sequences or sequences having at least 80% identity thereto, respectively:

i) heavy chain SEQ ID NO: 70, light chain SEQ ID NO: 71; or,
ii) heavy chain SEQ ID NO: 43, light chain SEQ ID NO: 46; or,
iii) heavy chain SEQ ID NO: 44, light chain SEQ ID NO: 46; or,
iv) heavy chain SEQ ID NO: 45, light chain SEQ ID NO: 46; or,
v) heavy chain SEQ ID NO: 42, light chain SEQ ID NO: 47; or,

vi) heavy chain SEQ ID NO: 43, light chain SEQ ID NO: 47; or,
vii) heavy chain SEQ ID NO: 44, light chain SEQ ID NO: 47; or,
viii) heavy chain SEQ ID NO: 45, light chain SEQ ID NO: 47; or,
ix) heavy chain SEQ ID NO: 56, light chain SEQ ID NO: 60; or,
x) heavy chain SEQ ID NO: 57, light chain SEQ ID NO: 60; or,
xi) heavy chain SEQ ID NO: 58, light chain SEQ ID NO: 60; or,
xii) heavy chain SEQ ID NO: 59, light chain SEQ ID NO: 60; or,
xiii) heavy chain SEQ ID NO: 56, light chain SEQ ID NO: 61; or,
xiv) heavy chain SEQ ID NO: 57, light chain SEQ ID NO: 61; or,
xv) heavy chain SEQ ID NO: 58, light chain SEQ ID NO: 61; or,
xvi) heavy chain SEQ ID NO: 59, light chain SEQ ID NO: 61; or,
xvii) heavy chain SEQ ID NO: 68, light chain SEQ ID NO: 71; or,
xviii) heavy chain SEQ ID NO: 69, light chain SEQ ID NO: 71; or,
xix) heavy chain SEQ ID NO: 42, light chain SEQ ID NO: 46; or,
xx) heavy chain SEQ ID NO: 68, light chain SEQ ID NO: 72; or,
xxi) heavy chain SEQ ID NO: 69, light chain SEQ ID NO: 72; or,
xxii) heavy chain SEQ ID NO: 70, light chain SEQ ID NO: 72.

8. The antibody or the antigen-binding fragment thereof according to any one of claims 1-7, being a murine antibody, a chimeric antibody, a humanized antibody, or a fully human antibody.

9. The antibody or the antigen-binding fragment thereof according to any one of claims 1-8, wherein the antibody or the antigen-binding fragment thereof is selected from the group consisting of a monoclonal antibody, a fusion protein, a multispecific antibody, a Fab fragment, a Fab' fragment, a F(ab')2 fragment, an Fd fragment, an Fv fragment, a dAb fragment, an isolated CDR region, an scFv, and a nanobody.

10. The antibody or the antigen-binding fragment thereof according to any one of claims 1-9, wherein the antibody or the antigen-binding fragment thereof is of the IgG1, IgG2, IgG3, or IgG4 type.

11. The antibody or the antigen-binding fragment thereof according to any one of claims 1-10, wherein the antibody or the antigen-binding fragment thereof exhibits one of or a combination of several of the following properties:

   (a) binding to Trop-2 with a $K_D$ value of 1.00E-08 M or less;
   (b) blocking binding of Trop-2 to the ligand;
   (c) exhibiting internalization activity in cells expressing Trop-2;
   (d) inducing ADCC against cells expressing Trop-2;
   (e) not binding to tumor cells that do not express Trop-2.

12. An antibody-drug conjugate, comprising the antibody or the antigen-binding fragment thereof according to any one of claims 1-11 linked to or conjugated to a therapeutic agent, preferably, the therapeutic agent is a cytotoxic drug, a radioisotope, an immunomodulator, or an antibody.

13. A pharmaceutical composition, comprising a pharmaceutically acceptable carrier, and i) the antibody or the antigen-binding fragment thereof according to any one of claims 1-11 or ii) the antibody-drug conjugate according to claim 12.

14. An isolated nucleic acid molecule, encoding the antibody or the antigen-binding fragment thereof according to any one of claims 1-11.

15. An expression vector comprising the nucleic acid molecule according to claim 14.

16. A host cell comprising the expression vector according to claim 15 or having the isolated nucleic acid molecule according to claim 14 integrated into genome thereof.

17. Use of the antibody or the antigen-binding fragment thereof according to any one of claims 1-11, the antibody-drug conjugate according to claim 12, or the pharmaceutical composition according to claim 13 for preparing a medicament for the treatment of a TROP-2-mediated disease.

18. The use according to claim 17, wherein the TROP-2-mediated disease is cancer.

**19.** A method for producing the antibody or the antigen-binding fragment thereof according to any one of claims 1-10, comprising culturing the host cell according to claim 16 under conditions enabling expression of the antibody or the antigen-binding fragment thereof and isolating the expressed antibody or antigen-binding fragment thereof.

FIG. 1

FIG. 2

FIG. 3

FIG. 4

FIG. 5

FIG. 6

FIG. 7

FIG. 8

## INTERNATIONAL SEARCH REPORT

| International application No. |
| --- |
| **PCT/CN2022/137192** |

### A. CLASSIFICATION OF SUBJECT MATTER

C07K16/30(2006.01)i;A61P35/00(2006.01)i;A61K39/395(2006.01)i

According to International Patent Classification (IPC) or to both national classification and IPC

### B. FIELDS SEARCHED

Minimum documentation searched (classification system followed by classification symbols)

C07K A61P A61K

Documentation searched other than minimum documentation to the extent that such documents are included in the fields searched

Electronic data base consulted during the international search (name of data base and, where practicable, search terms used)

CNTXT; WPABS; ENTXT; USTXT; JPTXT; KRTXT; DWPI; VEN; CJFD; ISI web of Science; PUBMED; 百度学术, BAIDU SCHOLAR; 百度, BAIDU; 读秀, DUXIU; 万方, WANFANG; CNKI; Genbank; EBI; ENA; STN; 中国专利生物序列检索, Chinese Patent Biological Sequence Retrieval System: 人TROP-2, trop-2, 滋养层细胞表面糖蛋白抗原, 肿瘤相关钙离子信号转导子2, TACSTD2, 表皮糖蛋白, EGP-1, 抗体, 单抗, antibody, 序列检索, sequence search

### C. DOCUMENTS CONSIDERED TO BE RELEVANT

| Category* | Citation of document, with indication, where appropriate, of the relevant passages | Relevant to claim No. |
| --- | --- | --- |
| A | CN 104053672 A (RINAT NEUROSCIENCE CORP.) 17 September 2014 (2014-09-17) claims 1-41 | 1-19 |
| A | CN 107406508 A (ABRUZZO THERANOSTIC S.R.L.) 28 November 2017 (2017-11-28) claims 1-27, and sequence table | 1-19 |
| A | CN 104114580 A (LIVTECH INC.) 22 October 2014 (2014-10-22) claims 1-44, and sequence table | 1-19 |
| A | CN 112646038 A (MABWELL (SHANGHAI) BIOTECHNOLOGY CO., LTD.) 13 April 2021 (2021-04-13) abstract, and claims 1-16 | 1-19 |
| A | WO 2020228604 A1 (JIANGSU HANSOH PHARMACEUTICAL GROUP CO., LTD. et al.) 19 November 2020 (2020-11-19) claims 1-25, and sequence table | 1-19 |
| A | US 2014377287 A1 (IMMUNOMEDICS, INC.) 25 December 2014 (2014-12-25) claims 1-56 | 1-19 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **15 February 2023** | **27 February 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.

**PCT/CN2022/137192**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

EP 4 438 627 A1

**INTERNATIONAL SEARCH REPORT**
**Information on patent family members**

International application No.

**PCT/CN2022/137192**

| Patent document cited in search report | | | Publication date (day/month/year) | Patent family member(s) | | | Publication date (day/month/year) |
|---|---|---|---|---|---|---|---|
| CN | 104053672 | A | 17 September 2014 | RU | 2014116406 | A | 20 December 2015 |
| | | | | US | 2014357844 | A1 | 04 December 2014 |
| | | | | US | 9399074 | B2 | 26 July 2016 |
| | | | | CA | 2954166 | A1 | 16 May 2013 |
| | | | | WO | 2013068946 | A2 | 16 May 2013 |
| | | | | WO | 2013068946 | A3 | 25 July 2013 |
| | | | | KR | 20140091040 | A | 18 July 2014 |
| | | | | JP | 2016104751 | A | 09 June 2016 |
| | | | | JP | 6157575 | B2 | 05 July 2017 |
| | | | | US | 2016257746 | A1 | 08 September 2016 |
| | | | | US | 10100114 | B2 | 16 October 2018 |
| | | | | CA | 2854720 | A1 | 16 May 2013 |
| | | | | CA | 2854720 | C | 18 December 2018 |
| | | | | BR | 112014011331 | A2 | 25 April 2017 |
| | | | | IL | 232426 | A0 | 30 June 2014 |
| | | | | TW | 201333036 | A | 16 August 2013 |
| | | | | TWI | 495644 | B | 11 August 2015 |
| | | | | SA | 112330988 | B1 | 22 July 2015 |
| | | | | CO | 7010785 | A2 | 31 July 2014 |
| | | | | US | 2019002559 | A1 | 03 January 2019 |
| | | | | US | 11091544 | B2 | 17 August 2021 |
| | | | | AR | 088693 | A1 | 25 June 2014 |
| | | | | HK | 1201851 | A1 | 11 September 2015 |
| | | | | MX | 2014005728 | A | 30 May 2014 |
| | | | | JP | 2014534233 | A | 18 December 2014 |
| | | | | JP | 5936702 | B2 | 22 June 2016 |
| | | | | US | 2013122020 | A1 | 16 May 2013 |
| | | | | US | 8871908 | B2 | 28 October 2014 |
| | | | | JP | 2017141249 | A | 17 August 2017 |
| | | | | JP | 6475277 | B2 | 27 February 2019 |
| | | | | EP | 2776470 | A2 | 17 September 2014 |
| | | | | ZA | 201403492 | B | 26 April 2017 |
| | | | | SG | 11201401699 | WA | 26 September 2014 |
| | | | | AU | 2012335205 | A1 | 29 May 2014 |
| CN | 107406508 | A | 28 November 2017 | JP | 2018500930 | A | 18 January 2018 |
| | | | | US | 2018002437 | A1 | 04 January 2018 |
| | | | | US | 10501555 | B2 | 10 December 2019 |
| | | | | EP | 3227340 | A1 | 11 October 2017 |
| | | | | EP | 3227340 | B1 | 17 June 2020 |
| | | | | CA | 2968330 | A1 | 09 June 2016 |
| | | | | AU | 2015357054 | A1 | 29 June 2017 |
| | | | | WO | 2016087651 | A1 | 09 June 2016 |
| CN | 104114580 | A | 22 October 2014 | ES | 2750358 | T3 | 25 March 2020 |
| | | | | EA | 201692042 | A1 | 30 January 2017 |
| | | | | EA | 035947 | B1 | 04 September 2020 |
| | | | | CA | 2855699 | A1 | 30 May 2013 |
| | | | | CA | 2855699 | C | 20 July 2021 |
| | | | | US | 2013344509 | A1 | 26 December 2013 |
| | | | | US | 9427464 | B2 | 30 August 2016 |
| | | | | AU | 2012341450 | A1 | 08 May 2014 |

Form PCT/ISA/210 (patent family annex) (July 2022)

**INTERNATIONAL SEARCH REPORT**
Information on patent family members

International application No.

**PCT/CN2022/137192**

| Patent document cited in search report | | | Publication date (day/month/year) | Patent family member(s) | | | Publication date (day/month/year) |
|---|---|---|---|---|---|---|---|
| | | | | AU | 2012341450 | B2 | 30 March 2017 |
| | | | | NZ | 623464 | A | 27 May 2016 |
| | | | | JPWO | 2013077458 | A1 | 27 April 2015 |
| | | | | JP | 6043725 | B2 | 14 December 2016 |
| | | | | MX | 2014006037 | A | 22 September 2014 |
| | | | | MX | 362720 | B | 31 October 2018 |
| | | | | CA | 3027417 | A1 | 30 May 2013 |
| | | | | BR | 112014012119 | A2 | 27 October 2020 |
| | | | | WO | 2013077458 | A1 | 30 May 2013 |
| | | | | EP | 3521313 | A1 | 07 August 2019 |
| | | | | EP | 3521313 | B1 | 27 July 2022 |
| | | | | ES | 2927090 | T3 | 02 November 2022 |
| | | | | EA | 201491006 | A1 | 30 October 2014 |
| | | | | EA | 035953 | B1 | 04 September 2020 |
| | | | | US | 2016333110 | A1 | 17 November 2016 |
| | | | | US | 10202461 | B2 | 12 February 2019 |
| | | | | EP | 2799452 | A1 | 05 November 2014 |
| | | | | EP | 2799452 | A4 | 15 July 2015 |
| | | | | EP | 2799452 | B1 | 21 August 2019 |
| | | | | KR | 20140102677 | A | 22 August 2014 |
| | | | | KR | 101972625 | B1 | 16 August 2019 |
| CN | 112646038 | A | 13 April 2021 | None | | | |
| WO | 2020228604 | A1 | 19 November 2020 | TW | 202108623 | A | 01 March 2021 |
| US | 2014377287 | A1 | 25 December 2014 | US | 9770517 | B2 | 26 September 2017 |

Form PCT/ISA/210 (patent family annex) (July 2022)

## REFERENCES CITED IN THE DESCRIPTION

**Patent documents cited in the description**

- CN 105849126 A
- US 5225539 A

- DD 120303

**Non-patent literature cited in the description**

- **E. MEYERS ; W. MILLER.** *Comput. Appl. Biosci.,* 1988, vol. 4, 11-17
- **NEEDLEMAN ; WUNSCH.** *J. Mol. Biol.,* 1970, 484-453
- Methods in Molecular Biology. Humana Press
- **SAMBROOK et al.** Molecular Cloning: A Laboratory Manual. 1989
- Current Protocols in Immunology. 1991
- **C. A. JANEWAY ; P. TRAVERS.** *Immunobiology,* 1997
- **P. FINCH ; 1997.** *Antibodies*
- Antibodies: a practical approach. IRL Press, 1988
- Monoclonal antibodies: a practical approach. Oxford University Press, 2000
- **C. BARBAS III et al.** Phage display: a laboratory manual. Cold Spring Harbor Laboratory Press, 2001

- **E. HARLOW ; D. LANE.** Using antibodies: a laboratory manual. Cold Spring Harbor Laboratory Press, 1999
- **KETTLEBOROUGH CA et al.** *European Journal of Immunology,* 1993, vol. 23, 206-211
- **STREBE N et al.** *Antibody Engineering,* 2010, vol. 1, 3-14
- **AL-LAZIKANI, B. ; CHOTHIA, C. ; LESK, A. M.** *J. Mol. Biol.,* 1997, vol. 273 (4), 927
- **CHOTHIA, C. et al.** *J Mol Biol,* 05 December 1985, vol. 186 (3), 651-63
- **CHOTHIA, C. ; LESK, A. M.** *J. Mol. Biol.,* 1987, vol. 196, 901
- **CHOTHIA, C. et al.** *Nature,* 21 December 1989, vol. 342 (6252), 877-83